# EUROPÄISCHE PATENTSCHRIFT

(11) **EP 0 641 184 B1**
(45) Veröffentlichungstag und Bekanntmachung des Hinweises auf die Patenterteilung: **03.01.1996**
(21) Anmeldenummer: 93912757.7
(22) Anmeldetag: 21.05.1993
(51) Int. Cl.: A61F 5/04

(54) **VORRICHTUNG ZUR UMSCHLIESSENDEN FIXIERUNG VON KÖRPEREXTREMITÄTEN**
EXTERNAL FIXING DEVICE FOR EXTREMITIES OF BODIES
DISPOSITIF DE FIXATION EXTERNE D'EXTREMITES DE CORPS

(30) Priorität: 22.05.1992 DE 4217061
(43) Veröffentlichungstag der Anmeldung: 08.03.1995
(73) Patentinhaber: Habermeyer, Peter, Dr., D-70184 Stuttgart (DE); HASSLER, Andreas, D-82166 Gräfelfing (DE)
(72) Erfinder: Habermeyer, Peter, Dr., D-70184 Stuttgart (DE); HASSLER, Andreas, D-82166 Gräfelfing (DE)
(74) Vertreter: Richter, Joachim, Dipl.-Ing.
(86) Internationale Anmeldenummer: EP9301283
(87) Internationale Veröffentlichungsnummer: WO9324081

(56) Entgegenhaltungen:
- EP-A- 0 355 930
- EP-A- 0 418 752
- WO-A-92/04880
- DE-U- 8 716 069

## Beschreibung

Die Erfindung betrifft eine Vorrichtung zur umschließenden Fixierung von Extremitäten und Extremitätenbereichen, insbesondere zur Behandlung von Extremitätenfrakturen im Bereich von Unterschenkel und Oberschenkel, bestehend aus einem Schalenkörper aus einem Schalenteil oder aus zwei gegeneinander verspannbaren L-förmigen Schalenteilen mit einem etwa halbkreisförmigen Querschnittsprofil, die die zu umschließende Extremität umgeben, wobei zwischen den Schalenteilen und der Extremität mindestens ein vakuierbares Manschettenkissen vorgesehen ist, in dessen zwischen den beiden Kissenwänden gelegenem Innenraum eine Vielzahl von relativ zueinander beweglichen Füllkörpern vorgesehen ist, wobei das Manschettenkissen eine Mehrzahl von untereinander verbundenen und mit Füllkörpern gefüllten vakuumdichten Bereichen aufweist, zwischen denen Stege und Inseln mit Luftdurchtrittsöffnungen ausgebildet sind.

Zur Behandlung von Extremitätenfrakturen werden üblicherweise Gipsverbände oder deren moderne Varianten in Form von Kunststoffverbänden angelegt. Das Anlegen derartiger Verbände erfordert nicht nur ein erhebliches Geschick und einen entsprechenden Aufwand für das Personal, sondern führt häufig auch dazu, daß durch Kanten oder Vorsprünge an der Innenseite des Gips- oder Plastikverbandes für den Patienten äußerst störende Druckstellen entstehen. Nach der Reposition der jeweiligen Fraktur ist der Patient der schmerzhaften Phase des Nachwickelns der Binden und des Wartens auf das Abbinden des Gipses ausgesetzt. Schließlich ist es bei den bekannten Gips- oder Plastikverbänden im Regelfall erforderlich, einen Wechsel dieses Gips- oder Plastikverbandes vorzunehmen, wenn es im Laufe der Fakturbehandlung zu einer Abschwellung der betreffenden Gliedmaßen kommt. Auch dies ist ein aufwendiger und für den Patienten wiederum unangenehmer Vorgang.

So ist durch die WO-A-9204880 eine Vorrichtung zur umschließenden Fixierung von Extremitäten und Extremitätenbereichen bekannt, die insbesondere zur Behandlung von Extremitätenfrakturen im Bereich von Unterschenkel, Oberschenkel, Unterarm und Oberarm vorgesehen ist. Diese Vorrichtung weist zumindest zwei gegeneinander verspannbare Schalenteile auf, die die zu schließende Extremität umgeben. Zwischen den Schalenteilen und der Extremität sind zwei formbare, vakuumdicht ausgebildete und mit zumindest einem Ventil versehene evakuierbare Kissen vorgesehen, in denen sich eine Vielzahl von relativ zueinander beweglichen, insbesondere kleinkörnigen Füllkörpern befindet.

Das Kissen besteht dabei aus einem oberen Schaftabschnitt und einem unteren Fußabschnitt. Auf diese Weise kann mit einem einzigen evakuierbaren Kissen sowohl der Unterschenkel als auch der Fuß eines Patienten umschlossen werden. Im mittleren Bereich des Fußabschnitts ist ein vakuumdichter Bereich als Fußbett ausgebildet, dessen Kontur sich beim Evakuieren des Kissens an das Fußgewölbe des Patienten anpaßt und auf diese Weise gleichzeitig den Fuß stützt. Unter dem Fuß sind ebenfalls Luftdurchtrittsöffnungen ausgebildet, die in Drainagekanäle münden und somit für eine Flüssigkeitsabfuhr aus dem Bereich unterhalb des Fußes sorgen.

Trotz der in dem Manschettenkissen ausgebildeten Luftdurchtrittsöffnungen wird keine ausreichende Belüftung im Wadenbereich und im Bereich des Fersenaufstandpunktes erreicht.

Die DE-U-8716069 beschreibt einen Sportschuh, insbesondere Lauf- und Tennisschuh, der aus einem Schuhoberteil und einem anvulkanisierten Schuhunterteil besteht, das wiederum aus einer Laufsohle, einer stoßdämpfenden, mit Luftkammern versehenen kombinierbaren Zwischensohle und einer innen angeordneten Innensohle aufgebaut ist, wobei die einzelnen Luftkammern der Zwischensohle über Verbindungskanäle untereinander verbunden sind. Im Fersenbereich des Schuhs sind mehrere Ansaugöffnungen vorgesehen, durch welche bei der alternativen Belastung der Zwischensohle der Innenkühlung des Schuhs dienende Kühlluft ansaugbar ist. Mit einem derart ausgebildeten Sportschuh soll der Vorteil erreicht werden, daß selbst bei intensiver Benutzung die innerhalb desselben auftretenden Temperaturen und/oder Feuchtigkeitswerte auf einer erträglichen Höhe gehalten werden sollen.

Der therapeutische Schuh nach der EP-A-0418752 weist neben einem Fußteil einen Schaft auf, der eine Winkelverstellbarkeit zum Fußteil zuläßt.

Bei denjenigen Fixierungsvorrichtungen, bei denen von zwei gegeneinander verspannbaren Schalenteilen ausgegangen wird, ergeben sich oftmals Anpassungsschwierigkeiten insbesondere durch unterschiedliche Wadenformgebungen und Wadenabmessungen. Die den Schalenkörper bildenden Schalenteile umgreifen abschnittsweise den Unterschenkel im rückwärtigen und im vorderen Bereich, und da diese Schalenteile eine gewisse Eigensteifigkeit aufweisen, ist aufgrund der unterschiedlilchen Wadenausgetaltung keine ausreichende Fomstabilität und Fixierung des Unterschenkels gegeben, zumal es Ziel sein sollte, eine anatomisch korrekte Stellung des Fußes zum Unterschenkel von 90° beizubehalten, d. h. die Normalwinkelstellung ist dabei erforderlich, um den Heilungsprozeß zu fördern. Wesentlich ist jedoch eine hohe Genauigkeit bei der Anpassung der Schalenteile und insbesondere des rückwärtigen Schalenteils des Schalenkörpers im Wadenbereich, die mit den bekannten Fixierungsvorrichtungen nicht erreicht wird.

Hinzu kommt, daß diese bekannten Gips- und Plastikverbände eine vorgegebene Winkelstellung zwischen dem Fuß und dem Unterschenkel voraussetzen, wobei der Normalwinkelstellung der Gelenke mit einem Winkel von 90° der Vorzug gegeben wird. Nach erfolgtem Anlegen des Gipsverbandes ist eine Veränderung der Winnkelstellung nicht möglich. Wird dagegen ein Operationsgips bei Achillessehnennaht angelegt, dann wird in der Weise vorgegangen, daß nach der Operation der Unterschenkel senkrecht aufgestellt und auf maximale Spitzfußstellung angehoben wird. Auch bei einem geschlossenen Spitzfußgips nimmt der Fuß zum Unterschenkel keine Normalwinkelstellung mit einem Winkel von 90° ein, sondern die Stellung des Fußes zum Unterschenkel erfolgt in einem Winkel von größer als 90°; oftmals in einem Winkel von 120°, so daß bei einem Spitzfußgips diese Winkelstellung des Fußes zum Unterschenkel vorgegeben und festgelegt ist.

Auch hier ist eine Änderung der Winkelstellung nicht möglich.

Es ist daher Aufgabe der vorliegenden Erfindung, eine Vorrichtung zur umschließenden Fixierung von Extremitäten und Extremitätenbereichen gemäß der eingangs genannten Art mit einer verbesserten Belüftung der umschlossenen Extremität sowie eine verbesserte Fixierung der Extremität zu schaffen. Es soll ferner die Möglichkeit einer Anpassung an die jeweilige Wade des Patienten gegeben sein. Außerdem soll die Vorrichtung universell für die verschiedensten Winkelstellungen des Fußes zum Unterschenkel einsetzbar sein, so daß insgesamt die Fixierung der Extremität verbessert wird.

Diese Aufgabe wird durch die im kennzeichnenden Teil des Anspruches 1 angegebenen Merkmale gelöst.

Die Schaffung eines mit Luftdurchtrittsöffnungen versehenen Inselbereichs im Bereich eines Muskelpolsters, beispielsweise im Bereich einer Wade eines zu umschließenden Unterschenkels, sorgt einerseits für eine verbesserte Belüftung dieses Bereichs der Extremität und andererseits für eine nahezu unmittelbare, nur durch die Wandung des Kissens unterbrochene Anlage dieses Muskelbereichs am zugeordneten Schalenteil. Das Fehlen der Füllkörper in diesem Inselbereich bewirkt dabei, daß die von den Füllkörpern ausgeübte thermische Isolationswirkung in diesem Bereich entfällt und so die Tendenz zum Schwitzen in diesem Muskelbereich reduziert ist. Außerdem werden dadurch das Gesamtvolumen des Kissens und der Umfang des Kissens in diesem Bereich verringert. Des weiteren wird bewirkt, daß die Ferse im wesentlichen unmittelbar auf einem zugeordneten Fußbett ohne Zwischenlage von Füllkörpern aufliegt, so daß eine verbesserte Vertikalabstützung und damit eine verbesserte Fixierung der Extremität gegeben ist. Auch die Belüftung der Ferse und der Fußunterseite ist hierdurch verbessert.

Die Ausgestaltung nach Anspruch 2 löst die zugrundegelegte Aufgabe ebenfalls auf vorteilhafte Weise, da die saugfähige Einlage für einen wirkungsvollen Feuchteabtransport von der Extremität sorgt, wobei die semipermeable Schicht der saugfähigen Einlage ein Zurückfließen der Feuchtigkeit von der saugfähigen Einlage zur Extremität verhindert.

Eine besonders wirkungsvolle Lösung der Aufgabe im Fußbereich ist im Anspruch 3 angegeben. Die Kombination der vertikalen Lüftungsdurchlässe mit den Querkanälen und den Längskanälen schafft eine wirkungsvolle Belüftung der Fußunterseite, wobei eine Walkbewegung im elastischen Fußbett für eine Pumpwirkung in den Lüftungsdurchlässen, den Längskanälen und den Querkanälen sorgt, so daß durch diese Pumpwirkung ein aktiver Luftaustausch im Bereich der Fußsohle, der Lüftungsdurchlässe und der Längs- und Querkanäle bewirkt wird. Dabei ist insbesondere die Ausgestaltung nach Anspruch 5 vorteilhaft, wodurch zusätzlich zum Luftaustausch an der Fußsohle auch ein Luftaustausch in den seitlichen Bereichen des Fußes erfolgt. Verbessert wird die Luftaustauschwirkung durch das im Anspruch 5 angegebene Merkmal, da auf diese Weise ein unmittelbarer Luftaustausch zwischen den Querkanälen und der Umgebungsluft erfolgen kann.

Die Ausgestaltung gemäß Anspruch 6 sorgt für eine wirkungsvolle Fixierung des Fußgelenkes im Bereich der Ferse und damit für einen festen, sicheren Halt der Extrmität in der Vorrichtung, wodurch gleichzeitig die die Pumpwirkung fördernde Walkbewegung beim Gehen unterstützt wird. Insbesondere vorteilhaft ist diese Ausführung jedoch für den Fall, daß die Vorrichtung zur umschließenden Fixierung der Extremität im Liegen angepaßt wird. Steht der Patient später auf, so kann durch ein Nachspannen des Spannelementes eine festere Unterstützung der Ferse herbeigeführt werden. Gleichzeitig wird aufgrund der Elastizität des Spannelementes eine leichte Federwirkung erzielt. Die besonders vorteilhafte Weiterbildung gemäß Anspruch 7 sorgt dabei für eine zusätzliche Sicherung der Schaftabdeckschale und bewirkt, daß diese bezüglich des Schalenteils im Bereich des Überganges zum Fußteil festgelegt ist.

Eine vorteilhafte Fixierung der Kissen ist im Anspruch 8 angegeben, wodurch ein im wesentlichen vertikales Verrutschen der Kissen verhindert ist.

Die Fixierungsvorrichtung selbst besteht dabei aus zwei eeine L-Form aufweisenden Schalenteilen, die zu einem Schalenkörper miteinander verspapnnbar sind und die ein etwa halbkreisförmiges Querschnittsprofil aufweisen, so daß der Unterschenkel des Patienten von beiden Seiten vermittels der beiden Schalenteile abschnittsweise umgriffen wird. Das rückwärtige Schalenteil des Schalenkörpers weist unter Ausbildung mindestens eines zungenartigen Abschnittes vom oberen Schalenteilenrand ausgehend schlitzförmige Durchbrechungen auf, die sich über einen Bereich in Schalenteillängsrichtung derart erstrecken, daß in dem rückwärtigen Schalenteil federnd-elastische und gegeneinander verschiebbare Wandabschnitte ausgebildet werden, wobei diese Abschnitte vermittels einer Einrichtung fixierbar bzw. verspannbar sind, damit eine hohe Formstabilität bei hoher Paßgenauigkeit erreicht wird. Dadurch, daß der rückwärtige Schalenteil des Schalenkörpers gegeneinander verschiebbare, federnd-elastische Wandabschnitte aufweist, ist dieser Schalenteil jeder Wadengröße und Abmessung anpaßbar, so daß eine Lagenfixierung des Unterschenkels des Patienten im Schalenkörper gewährleistet ist, und zwar unter Beibehaltung der Normalwinkelstellung zwischen Fuß und Unterschenkel. Die Einrichtung zur Fixierung der gegeneinander verschiebbaren und federnd-elastischen Wandabschnitte des rückwärtigen Schalenteils kann als Spanngurt ausgebildet sein, wobei jedoch auch andersartig ausgebildete Fixierungseinrichtungen eingesetzt werden können, so z. B. Klemmeinrichtungen u. dgl. Wesentlich ist dabei, daß in Anpassung an die jeweilige Wadenformgebung und Wadengröße des Unterschenkels des Patienten die Schalenteile des Schalenkörpers so fixiert sind, daß nicht nur eine Stabilisierung im rückwärtigen Bereich, sondern auch eine seitliche Stabilisierung erhalten wird.

Die Fixierungsvorrichtung ist ferner in der Weise ausgebildet, daß von den beiden, eine etwa L-Form aufweisenden Schalenteilen mit einem etwa halbkreisförmigen Querschnittsprofil und mit einem Schaft und einem Fußteil das rückwärtige Schalenteil ein zu dessen Schaftwinkel verstellbares Fußteil und in dem dem Fußteil gegenüberliegenden Bandbereich eine fensterartige Durchbrechung mit einem in diese eingesetzten und austauschbaren plattenförmigen Winkeladapter aufweist, der mit einer Eingrifföffnung versehen ist, in die zur Winkelfestlegung zwischen dem Fußteil und dem Schaft des rückwärtigen Schalenteiles ein Nocken eingreift, der zur Fixierung einenr vorbestimmten Winkelstellung an dem Fußteil des rückwärtigen Schalenteiles angeformt ist.

Dadurch, daß das rückwärtige Schalenteil des Schalenkörpers der Fixierungsvorrichtung zur Aufnahme eines plattenförmigen Winkeladapters ausgebildet ist, besteht in Verbindung mit dem an dem Fußteil des rückwärtigen Schalenteils angeordneten Nocken die Möglichkeit, das Fußteil zum Schaft in der jeweils erforderlichen Winkelstellung anzuordnen, festzustellen und zu halten, wobei die Winkelstellung des Fußteils vorgegeben wird durch die Lage und Anordnung der Eingrifföffnung in dem plattenförmigen Winkeladapter. Ist die Eingrifföffnung im unteren Bereich des Winkeladapters vorgesehen und greift in die so lagevorgegebene Eingrifföffnung der Nocken des Fußteils in diese Eingrifföffnung ein, dann wird die Normalwinkelstellung des Fußes zum unteren Gelenk, nämlich die Winkelstellung von 90° erhalten, wohingegen bei einer Anordnung der Eingrifföffnung für die Nocken im oberen Bereich des Winkeladapters die bei 120° liegende Spitzfußstellung des Fußes zum Unterschenkel erhalten wird. Ist dagegen der plattenförmige Winkeladapter mit einer schlitzförmigen, sich in etwa über die gesamte Länge des WInkeladapters erstreckenden Eingrifföffnung versehen, dann besteht die Möglichkeit, die verschiedensten Winkelstellungen des Fußes zu dem Unterschenkel des Patienten einstellen zu können. Ist darüber hinaus der an dem Fußteil angeformte Nocken mit einer Feststellschraube versehen, dann ist die MöÖglichkeit gegeben, den in einer bestimmten Winkelstellung eingestellten Fußteil zum Schaft hin festzustellen.

Dadurch, daß in der Wand des rückwärtigen Schaftteils der plattenförmige Winkeladapter in einer festerartigen Durchbrechung zweckmäßigerweise mittels Klemmsitz gehalten ist, ist ein müheloses Auswechseln von Winkeladaptern mit den verschiedensten Anordnungen und Lagen der Eingrifföffnungen möglich. Der weitere Vorteil besteht darin, daß auch Lageveränderungen, d. h. Winkelveränderungen des Fußteils nach einer bestimmten Behandlungszeit z. B. mühelos möglich sind. Es sind somit in einfachster Weise die verschiedensten Winkelstellungen fixierbar, was gegenüber den bekannten Gipsverbänden insofern besonders vorteilhaft ist, als die bekannten Gipsverbände nur die eine oder andere Winkelstellung berücksichtigen können, da nach Erhärten des Gipses keine Winkelveränderungen des Fußteils mehr vorgenommen werden können.

Eine weitere Ausgestaltung der Fixierungsvorrichtung sieht vor, daß von den beiden, eine etwa L-Form aufweisenden Schalenteilen mit einem etwa halbkreisförmigen Querschnittsprofil und mit einem Schaft und einem Fußteil, das vordere Schalenteil ein zu dessen Schaft winkelverstellbares Fußteil und in dem Bereich zwischen dem Fußteil und dem Schaft eine Winkeleinstelleinrichtung aufweist, vermittels der das Fußteil zum Schaft in einer vorgegebenen Winkelstellung arretierbar ist, wobei das rückwärtige Schalenteil ein zu dessen Schaft winkelverstellbares Fußteil oder ein zu dessen Schaft in einem vorgegebenen Winkel festgestelltes Fußteil aufweist.

Dadurch, daß das vordere Schalenteil des Schalenkörpers der Fixierungsvorrichtung zur Aufnahme eines plattenförmigen Winkeladapters als Winkeleinstelleinrichtung ausgebildet ist, ist die Möglichkeit gegeben, das Fußteil zum Schaft in der jeweils erforderlichen Winkelstellung anzuordnen und somit das Fußteil zum Schaft in dem jeweils erforderlichen Winkel festzustellen, wobei die Winkelstellung des Fußteils vorgegeben wird durch die Lage und Anordnung und Abmessung des Winkeladapters.

Bei einer bevorzugten Ausführungsform besteht die Winkeleinstelleinrichtung für das Fußteil zum Schaft des vorderen Schalenteils des Schalenkörpers der Fixierungsvorrichtung aus dem zwischen dem Fußteil und dem Schaft des vorderen Schalenkörpers angeordneten, dem Außenprofil des Schalenkörpers bzw. dem vorderen Schalenkörper entsprechend profilierten, plattenförmigen Winkeladapter, der mittels Rast- und Verriegelungseinrichtungen an dem Schaft und em Fußteil des vorderen Schalenkörpers lösbar gehalten ist. Dieser plattenförmige Winkeladapter weist nach einer Ausführungsform eine Breite auf, aufgrund der das Fußteil zu dem Schaft des vorderen Schalenkörpers eine Winkelstellung von 90° einnimmt, so daß bei eingesetztem Winkeladapter das Fußteil zu dem Schaft in einem Winkel von 90° steht. Nach einer weiteren Ausführungsform weist der plattenförmige Winkeladapter eine Breite auf, aufgrund der das Fußteil zu dem Schaft des vorderen Schalenkörpers eine Winkelstellung von 120° einnimmt. Auf diese Weise ist es möglich, die Normalwinkelstellung des Fußes zum unteren Gelenk, nämlich die Winkelstellung von 90° zu erhalten, wohingegen bei der Anordnung eines breiteren Winkeladapters die bei 120° liegende Spitzfußstellung des Fußes zum Unterschenkel eingestellt und erhalten werden kann.

Für den Einsatz unterschiedlich bemessener Winkeladapter weist das Fußteil und der Schaft des vorderen Schalenkörpers im Verbindungsbereich vom Fußteil zum Schaft eine fensterartige Durchbrechung auf, die sich bis in den Seitenbereich des Fußteils und des Schaftes erstreckt, so daß der in die fensterartige Durchbrechung eingesetzte plattenförmige Winkeladapter diesen offenen Bereich zwischen dem Fußteil und dem Schaft überbrückt, wodurch die Winkelstellung des Fußteiles zum Schaft erhalten wird.

Die Befestigung des Winkeladapters erfolgt bevorzugterweise vermittels feststehender und verdrehbarer Verriegelungshaken und unter Verwendung von profilierten Randbereichen, wobei die verdrehbaren Verriegelungshaken als Exzenter ausgebildet sind, so daß durch die verdrehbaren, exzentrisch ausgebildeten Verriegelungshaken der Winkeladapter über die Gegenteile, d. h. über den Fußteil und den Schaft des vorderen Schalenkörpers, gespannt wird, wodurch ein spielfreier Preßsitz erreicht wird. Auf diese Weise ist es möglich, mühelos durch Verwendung des entsprechend ausgebildeten Winkeladapters die eine oder andere erforderliche Winkelstellung zu erreichen.

Aufgrund dieser Ausgestaltung der Fixierungsvorrichtung ist es möglich, bei einem aus zwei gegeneinander verspannbaren Schalenteilen bestehenden Schalenkörper auch das vordere Schalenteil, bei dem das Fußteil zum Schaft eine vorgegebene, nicht veränderbare Winkelstellung einnehmen soll, der jeweils erforderlichen Winkelstellung und somit der durch das rückwärtige Schalenteil vorgegebenen Winkelstellung anzupassen. Der vordere Schalenkörper besteht hiernach aus dem Schaft, em Fußteil und dem zwischen beiden Teilen eingesetzten Winkeladapter, wodurch die Möglichkeit gegeben ist, von einer Spitzfußstellung - Winkelstellung 120° - die Fixierungsvorrichtung auf die Normalwinkelstellung - Winkelstellung 90° - zurückzustellen. Es ist somit eine Anpassungsfähigkeit des vorderen Schalenteiles an das rückwärtige Schalenteil gegeben. Im vorliegenden Fall werden lediglich zwei Winkeladaptertypen benötigt, nämlich einmal ein Winkeladapter, der die Einstellung des Winkels 90° und ein Winkeladapter, der eine Einstellung auf 120° ermöglicht.

Eine weitere Ausführungsform sieht vor, daß das zum Schaft verstellbare Fußteil des vorderen Schalenteiles des Schalenkörpers der Fixierungsvorrichtung über einen quer zur Schaftlängsrichtung verlaufenden, ziehharmonikabalgartig ausgebildeten Abschnitt mit einer Anzahl von gefalteten, filmscharnierartig ausgebildeten und miteinander verbundenen Materialstreifen verbunden ist, wobei die das Fußteil und den Schaft miteinander verbindenden, seitlich an dem Schalenkörper ausgebildeten Schwenkgelenke mit Winkelarretierungseinrichtungen versehen sind. Die Verschwenkbarkeit und somit die Winkelveränderbarkeit des Fußteils zum Schaft wird mittels einer Vielzahl von parallel zueinander angeordneten Filmscharnieren erreicht, wobei dann die Feststellung des Fußteiles zum Schaft in den Winkelstellungen von 90° und 120° über die Schwenkgelenke erfolgt. Der Gelenkbereich zwischen dem Fußteil und dem Schaft des vorderen Schalenkörpers wird hier durch einen materialabschnitt erreicht, der eine Breitenveränderbarkeit ermöglicht.

Mit allen Ausgestaltungen einer Winkelverstellbarkeit wird der Vorteil erreicht, daß auch Lageveränderungen, d. h. Winkelveränderungen, des Fußteiles nach einer bestimmten Behandlungszeit z. B. mühelos möglich sind. Es sind somit in einfachster Weise die verschiedensten Winkelstellungen fixierbar, was gegenüber den bekannten Gipsverbänden insofern besonders vorteilhaft ist, als die bekannten Gipsverbände nur die eine oder andere Winkelstellung einnehmen können, da nach Erhärten des Gipses keine Winkelveränderungen des Fußteiles mehr vorgenommen werden können. Es ist somit eine Fixierungsvorrichtung geschaffen, die auch bei der Verwendung eines aus zwei Schalenteilen bestehenden Schalenkörpers mit einem rückwärtigen Schalenteil, dessen Fußteil zum Schaft winkelveränderbar ist, universell in den verschiedensten Winkelstellungen des Fußes zum Unterschenkel, bevorzugt bei einer 90° oder 120° Winkelstellung, einsetzbar ist.

Weitere vorteilhafte Ausgestaltungen der Erfindung sind Gegenstand der Unteransprüche.

Ausführungsbeispiele der Erfindung werden nachstehend anhand der Zeichnung näher erläutert. Es zeigen
Fig. 1 eine vorteilhafte Ausgestaltung eines Manschettenkissens nach der Erfindung,
Fig. 2 eine Draufsicht auf ein teilweise abgebrochen gezeigtes Fußbett,
Fig. 3 einen Längsschnitt durch das Fußbett nach Fig. 2 eintlang der Linie III-III,
Fig. 4 eine schwenkbar ausgebildete Zwischensohle,
Fig. 5 eine teilweise geschnittene Darstellung einer erfindungsgemäßen Vorrichtung zum Umschließen eines Fußgelenkes,
Fig. 6 eine Seitenansicht der Fixierungsvorrichtung aus einem Schalenkörper mit zwei L-förmigen Schalenteilen,
Fig. 7 die Fixierungsvorrichtung teils in einer Seitenansicht, teils in einem senkrechten Längsschnitt,
Fig. 8 in einer Seitenansicht die beiden L-förmigen Schalenteile des Schalenkörpers vor deren Verspannung,
Fig. 9 eine Seitenansicht der Schalenkörper,
Fig. 10 einen waagerechten Schnitt gemäß Linie X-X in Fig. 7,
Fig. 11 eine Seitenansicht des rückwärtigen Schalenteiles,
Fig. 12 eine Rückansicht des rückwärtigen Schalenteiles,
Fig. 13 eine schaubildliche Ansicht des rückwärtigen Schalenteils der aus zwei L-förmigen Schalenteilen bestehenden Fixierungsvorrichtung,
Fig. 14 eine Seitenansicht der Fixierungsvorrichtung aus einem Schalenkörper mit zwei L-förmigen Schalenteilen,
Fig. 15 die Fixierungsvorrichtung teils in einer Seitenansicht, teils in einem senkrechten Längsschnitt,

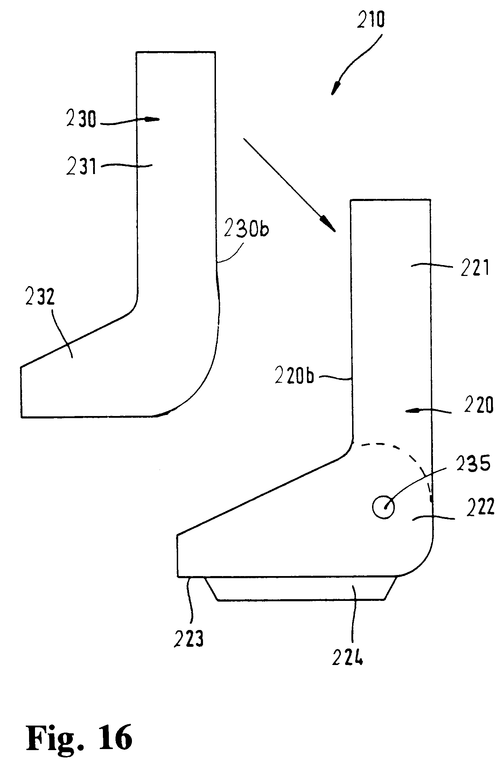

Fig. 16 in einer Seitenansicht die beiden L-förmigen Schalenteile des Schalenkörpers vor deren Verspannung,
Fig. 17 in einer Seitenansicht die aus einem plattenförmigen Winkeladapter und einem in diesen eingreifenden Nocken bestehende Winkeleinstelleinrichtung,
Fig. 18 in einer Vorderansicht den plattenförmigen Winkeladapter,
Fig. 19 eine Ansicht von oben auf den Winkeladapter gemäß Fig. 18,
Fig. 20 eine schematische Ansicht eines Winkeladapters zur Einstellung eines der Normalwinkelstellung von 90° entsprechenden Winkels,
Fig. 21 eine schematische Ansicht eines Winkeladapters zur Einstellung eines der Spitzfußstellung von 120° entsprechenden Winkels,
Fig. 22 eine schematische Ansicht eines Winkeladapters mit einer Winkeleinstellmöglichkeit über einen größeren Bereich,
Fig. 23 eine schaubildliche Ansicht des vorderen Schalenteiles der aus zwei L-förmigen Schalenteilen bestehenden Fixierungsvorrichtung mit eingesetztem Winkeladapter,
Fig. 24 eine Seitenansicht der Fixierungsvorrichtung aus einem Schalenkörper mit zwei L-förmigen Schalenteilen,
Fig. 25 die Fixierungsvorrichtung gemäß Fig. 23 teils in einer Seitenansicht, teils in einem senkrechten Längsschnitt,
Fig. 26 in einer Seitenansicht die beiden L-förmigen Schalenteile des Schalenkörpers vor deren Verspannung,
Fig. 27 in einer schaubildlichen Ansicht einen Winkeladapter für eine Winkelstellung von 90° zwischen dem Fußteil und dem Schaft des vorderen Schalenkörpers,
Fig. 28 in einer schaubildlichen Ansicht einen Winkeladapter für eine Winkelstellung von 120° zwischen dem Fußteil und dem Schaft des vorderen Schalenkörpers,
Fig. 29 einen Schnitt gemäß Linie XXIX-XXIS in Fig. 27, und
Fig. 30 in einer vergrößerten Wiedergabe den Verbindungsabschnitt A zwischen dem Winkeladapter und der Wandfläche des Schaftes des vorderen Schalenkörpers.

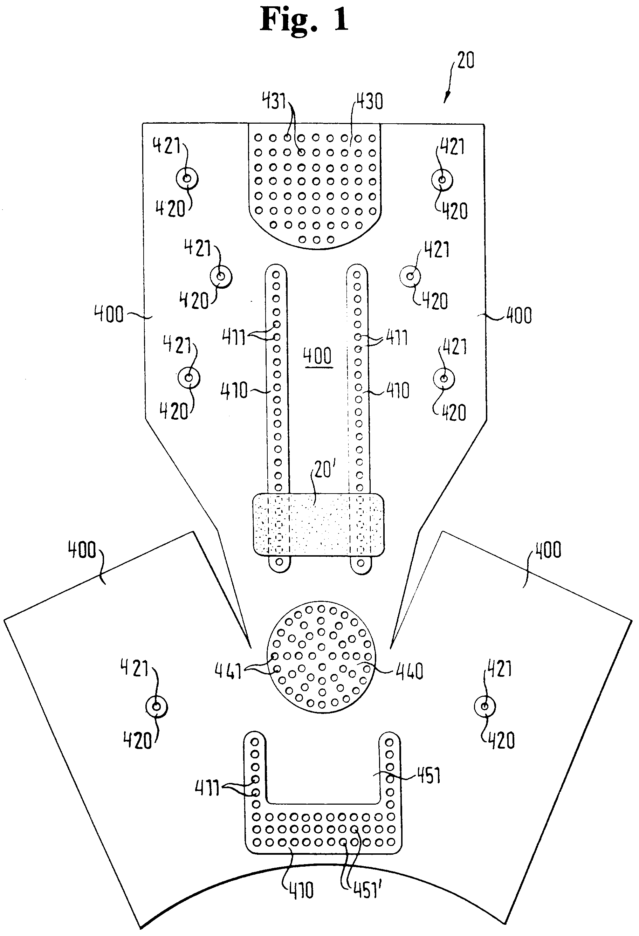

In Fig. 1 ist ein flächiges, evakuierbares Kissen gezeigt, wobei das Ventil auf der nicht einsehbaren Rückseite liegt. Das Kissen besitzt eine Merhzahl von untereinander verbundenen und mit Füllkörpern gefüllten vakuumdichten Bereichen 400, zwischen denen Stege 410 und Inseln 420 ausgebildet sind. Die Stege 410 und die Inseln 420 sind mit Luftdurchtrittsöffnungen 411, 421 versehen. Die Stege 410 verlaufen dabei vorzugsweise zick-zack-förmig oder gerade in Richtung der Längserstreckung des Kissens 20.

Im evakuierten Zustand der Kissen bilden die Stege Be- und Entlüftungskanäle. Weitere Be- und Entlüftungskanäle werden durch beim Evakuieren des Kissens entstehende negative Falten im Kissen gebildet, so daß sich auch zwischen den Inseln 420 und den Stegen 410 Kanäle ausbilden. Durch diese Kanäle und die Luftdurchtrittsöffnungen 411, 421 kann ein Luftaustausch zwischen der Oberfläche des umschlossenen Körperteils und der Außenseite des dieses Körperteil umschließenden Kissens stattfinden. Auch die durch die Füllkörper bedingte unebene Oberfläche des Kissens im evakuierten Zustand sorgt für die Bildung kleinster Be- und Entlüftungskanäle.

Im oberen Abschnitt des Manschettenkissens 20, im Bereich der Wade eines zu umschließenden Unterschenkels, ist ein Inselbereich 430 vorgesehen, der Luftdurchtrittsöffnungen 431 aufweist. Im Bereich der Ferse der zu umschließenden Extremität ist im Kissen 20 ein Inselbereich 440 vorgesehen, der den Fersenaufstandspunkt markiert und mit Luftdurchtrittsöffnungen 441 versehen ist.

Das Kissen 20 ist in den Schalenteilen derart angeordnet, daß zumindest die von den Stege 210 gebildeten Kanäle, vorzugsweise aber auch die Inseln 420, 430 und 440, im Bereich der Luftdurchlässe 70, 71 des zugehörigen Schalenteils 11, 12 gelegen sind. Dadurch wird der Luftaustausch zwischen den umschlossenen Körperteilen und der Außenseite der Vorichtung gewährleistet.

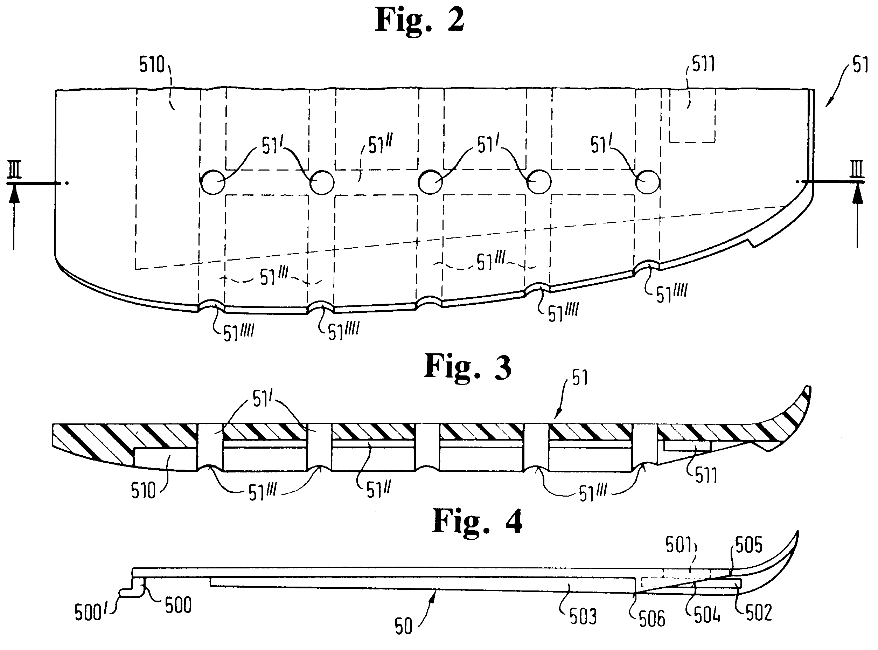

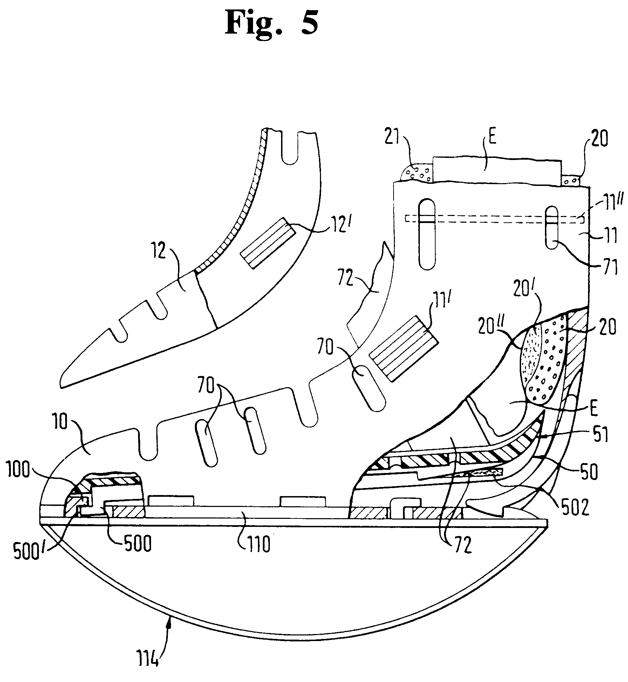

Fig. 2 zeigt die Draufsicht auf ein Fußbett 51, das oberhalb einer Zwischensohle 50 (Fig. 5) angeordnet ist und aus einem elastischen, stoßdämpfenden Material besteht. Das Fußbett 51 besitzt vertikale Lüftungsdurchlässe 51', die sich von der Oberseite des Fußbettes zu dessen Unterseite erstrecken (Fig. 3). An der Unterseite des Fußbettes 51 münden die vertikalen Lüftungsdurchlässe 51' in Längskanäle 51'' bzw. in Querkanäle 51'''. In Fig. 2 ist eine Ausführungsform gezeigt, bei er die vertikalen Lüftungsdurchlässe 51' jeweils an Kreuzungspunkten von Querkanälen 51''' und Längskanälen 51'' münden. Ein Teil der vertikalen Lüftungsdurchlässe 51' kann aber auch ausschließlich in Längskanäle 51'' münden, während ein anderer Teil der vertikalen Lüftungsdurchlässe 51' ausschließlich in Querkanäle 51''' mündet.

In Fig. 2 ist zu erkennen, daß die Querkanäle 51''' an den Außenseiten des Fußbettes 51 im wesentlichen nach oben verlaufen und sich nach oben öffnen, was durch 51'''' angezeigt ist.

An der Unterseite des Fußbettes 51 ist eine zentrale, sich im wesentlichen nach vorne erweiternde Ausnehmung 510 vorgesehen. Diese Ausnehmung dient zur Aufnahme der in Fig. 4 gezeigten Zwischensohle 50. Im Fersenbereich des Fußbettes 51 steht ein vorzugsweise rechteckiger Vorsprung 511 nach unten in die Ausnehmung 510 hervor. Der Vorsprung 511 ist zum Eingriff in eine passende Ausnehmung 501 im Fersenbereich der Zwischensohle 50 ausgelegt. Durch diese Formschlußverbindung wird zusätzlich zur trapezförmigen Ausgestaltung der Ausnehmung 510 und zur angepaßt trapezförmigen Ausbildung der Zwischensohle 50 eine weitere Formschlußverbindung zwischen der Zwischensohle 50 und dem Fußbett 51 geschaffen.

Die Zwischensohle 50 ist an ihrem vorderen Ende mit nach unten vorstehenden Gelenkhaken 500 versehen, deren freies Ende 500' sich nach vorn erstreckt. Im Bereich der Ferse ist die Zwischensohle 50 mit einem in Querrichtung verlaufenden Schlitz 502 versehen, der, wie in Fig. 5 zu sehen ist, zum Durchführen eines Spannbandes 72 dient. Seitlich neben dem nur in einem mittleren, nach unten hervorstehenden Abschnitt 503 der Zwischensohle 50 vorgesehenen Schlitz 502 sind schräg verlaufende Umlenkkanten 504 ausgebildet, die von einem hinteren, höheren Punkt 505 zu einem vorderen, niedriger gelegenen Punkt 506 verlaufen. Auf diese Weise wird das druch den Schlitz 502 geführte Spannband 72 schräg nach vorn umgelenkt, wie dies in Fig. 5 zu erkennen ist.

Im montierten Zustand greift der vordere Haken 500 der Zwischensohle 50 mit seinem freien Endabschnitt 500' unter einen Gegenhaken 100 in der mit einem Laufsohlenteil 114 verbundenen Grundfläche 110 des Fußteils 10. Diese Verhakung der Zwischensohle 50 im Fußteil 10 schafft eine um eine Querachse schwenkbare Lagerung für die Zwischensohle 50.

Im Fersenbereich wird die Zwischensohle 50 durch das beidseitig mit seinen freien Enden aus dem Schaftteil 11 herausgeführte Spannband 72 gehalten, das über der auf das Schaftteil aufgesetzten Schaftabdeckschale im Bereich des Fußgelenkes zusammengespannt wird und die Schaftabdeckschale 12 gegen den Schaftteil 11 drückt, wodurch die vom Spannband 72 übertragenen Haltekräfte für die Zwischensohle 50 über die Schaftabdeckschale 12 auf das Schaftteil 11 abgestützt werden. Das Spannband 72 wirkt aufgrund seiner ihm eigenen Elastizität als Feder-Dämpfer-System für den auf der Zwischensohle 50 stehenden Fuß.

Im Bereich des Übergangs zum Fußteil 10 am Schaftteil 11 vorgesehene Verrastungseinrichtungen 11' wirken mit an der Innenseite der Schaftabdeckschale 12 vorgesehenen Verrastungseinrichtungen 12' derart zusammen, daß beim Festspannen des Spannbandes 72 die Verrastungseinrichtungen 11' und 12' miteinander verhaken und so ein Nach-Vorne-Wandern der Schaftabdeckschale 12 verhindern.

Seitliche Belüftungsöffnungen 70 im Fußteil 10 sind mit den Querkanälen 51''' ausgerichtet und befinden sich insbesondere im Bereich der seitlich nach oben verlaufenden Abschnitte 51'''' der Querkanäle 51''', so daß ein intensiver Luftaustausch zwischen der Unterseite des Fußbettes 51 und der Umgebungsluft stattfinden kann.

Auf der Innenseite des Schaftteils 11 sind im wesentlichen horizontal verlaufende Rippen 11'' vorgesehen, die nach innen vorspringen und mit denen sich die Kissen 20 und 21 beim Evakuieren verhaken, so daß ein vertikales Verrutschen der Kissen 20 und 21 verhindert ist.

Im Bereich der Achillessehne und vorzugsweise auch im Fußbereich ist das Kissen 20 mit einer augfähigen Einlage 20' versehen, die an ihrer zur Extremität weisenden Seite eine für Dampf und Feuchtigkeit semipermeable Schicht besitzt. Auf diese Weise kann von der Extremität abgegebener Schweißschnell von der Haut weg in die saugfähige Einlage 20' transportiert werden, von wo die Feuchtigkeit über den Luftaustausch durch die Luftdurchtrittsöffnungen 411 hindurch an die Umgebung abgegeben wird.

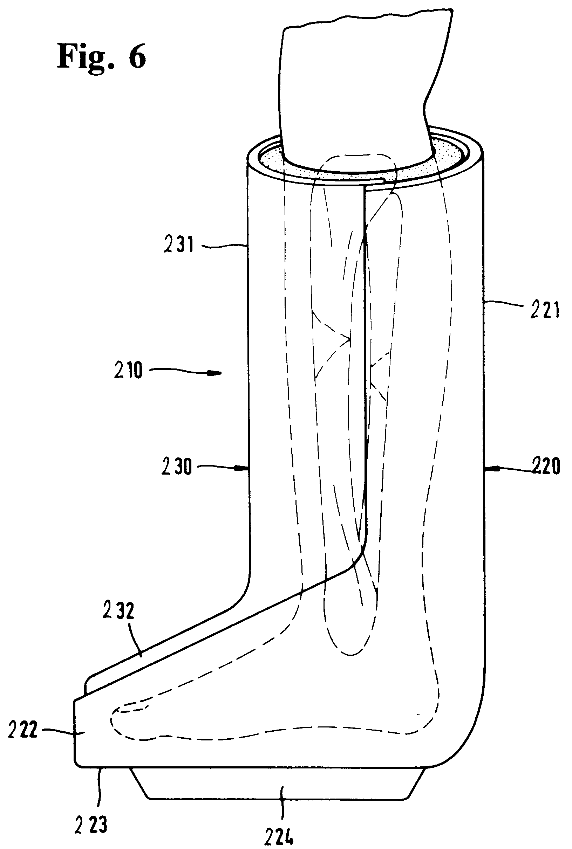

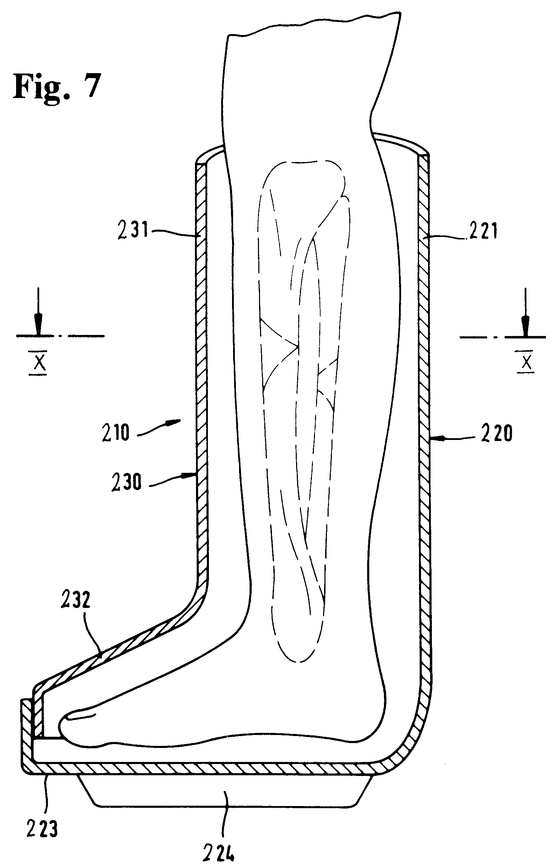

Eine Vorrichtung zur umschließenden Fixierung von Extremitäten ist in Fig. 6 und 7 als Schalenkörper 210 für den Fuß-/Unterschenkel-Bereich eines Patienten dargestellt. Der Schalenkörper 210 besteht aus zwei gegeneinander verspannbaren Schalenteilen, nämlich einem rückwärtigen Schalenteil 220 und einem vorderen Schalenteil 230, wobei beide Schalenteile 220, 230 in etwa eine L-Form aufweisen. Jedes Schalenteil 220 bzw. 230 in etwa eine L-Form aufweisen. Jedes Schalenteil 220 bzw. 230 besteht aus einem Schaft 221 bzw. 231 und einem Fußteil 222 bzw. 232, wobei das Fußteil 222 des Schalenteils 220 mit seinem Schaft 221 über seitliche Schwenkgelenke 235 miteinander verbunden sein kann. Sowohl die beiden Schalenteile 220, 230 als auch die Gelenke bestehen zweckmäßigerweise aus Kunststoff.

Das Fußteil 222 und der Schaft 221 des rückwärtigen Schalenteils 220 sind schalenförmig nach oben und nach vorn offen ausgebildet, so daß eine Aufnahmewanne für den Fuß und den Unterschenkel geschaffen ist. An der Unterseite des Fußteils 222 ist eine Laufsohle 223 vorgesehen, deren äußere Gestalt an die Abrollbewegung des Fußes beim Gehen angepaßt ist. Hierzu ist die Laufsohle 223 als Gehwiege ausgebildet oder mit einem Gehstollen 224 versehen.

Das Fußteil 232 und der Schaft 231 des vorderen Schalenteils 230 des Schalenkörpers 210 sind in Richtung zum Fuß und zum Unterschenkel schalenförmig ausgebildet, wobei dieses Schalenteil 230 so ausgebildet ist, daß das vordere Schalenteil 230 in das rückwärtige Schalenteil 220 derart einsetzbar ist, daß die seitlichen Wandabschnitte des Schalenteils 220 die seitlichen Wandabschnitte des Schalenteils 230 abschnittsweise übergreifen.

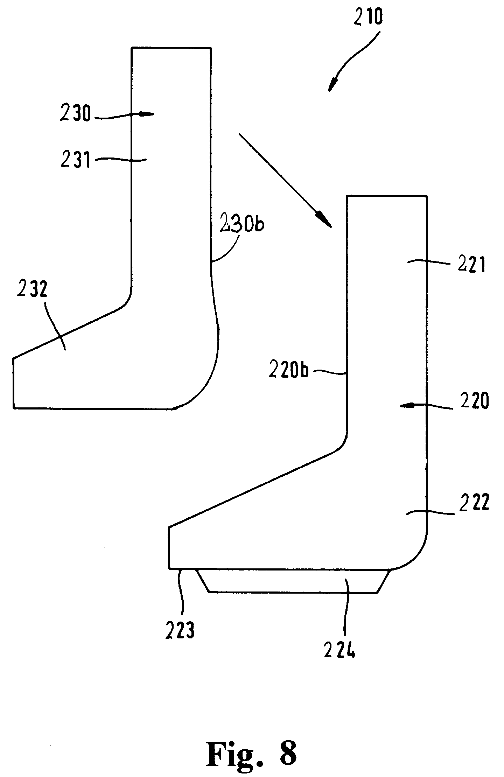

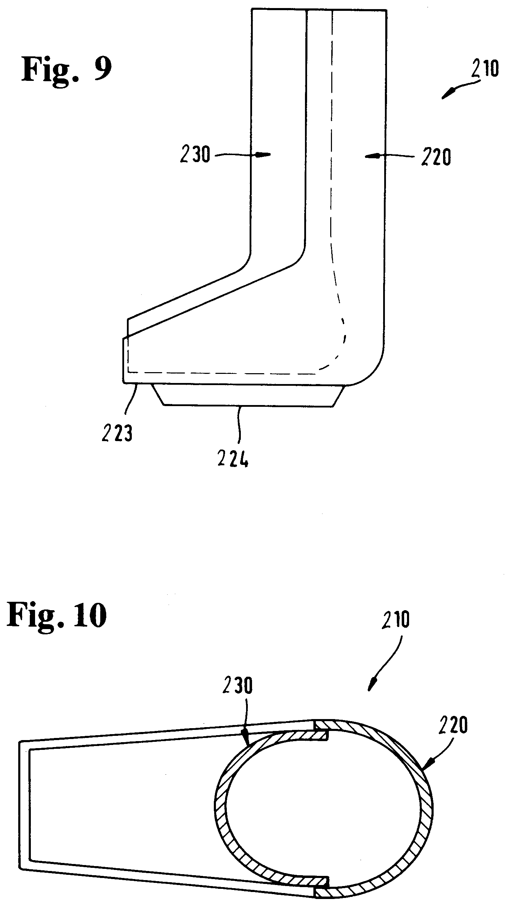

Sowohl das schalenteil 220 als auch das Schalenteil 230 weisen einen etwa halbkreisförmigen Querschnitt auf, so daß bei ineinandergesetzten Schalenteilen ein abschnittsweises Übergreifen der Seitenwandabschnitte der beiden Schalenteile 220, 230 erfolgt (Fig. 8, 9 und 10). Um den Unterschenkel und auch den Fuß seitlich umgreifen zu können, weisen die Schalenteile 220, 230 eine gewisse Elastizität auf, wobei zweckmäßigerweise die Elastizität der Seitenwandungen der beiden Schalenteile 220, 230 von ihren mittigen Bereichen der Schalenteile zu ihren vorderen freien Rändern 220b bzw. 230b eines jeden Schalenteils zunimmt.

Zwischen den beiden gegeneinander verspannbaren Schalenteilen 220, 230 und der Extremität ist ein verformbares, vakuumdicht ausgebildetes und mit zumindest einem Ventil versehenes Kissen 20 vorgesehen, das aus einer Blase besteht, in der eine Vielzahl von relativ zueinander beweglichen Füllkörpern vorgesehen ist. Das eng und passend an dem zu behandelnden Gliedabschnitt anlegbare Kissen wird bei einer innerhalb kurzer Zeit durchführbaren Evakuierung in der während des Anlegevorganges geschaffenen Form hart und formstabil, so daß in Verbindung mit den harten Schalenteilen eine sehr feste, an die jeweilige Extremität gut anmodellierte Hülse entsteht, die im Bereich der Haut keinerlei Druckstellen erzeugen kann, da bei der Verfestigung dieses Gebildes kein radial nach innen gerichteter Druck entseht und an der Innenseite auch keine Kanten oder Vorsprünge gebildet werden können. Anstelle eines derartigen Manschettenkissens kann auch ein anderweitiges Polster, Strumpf od. dgl. zur Zwischenraumausfüllung eingesetzt werden.

Die an den Längsrandbereichen von Fußteil und Schaft zweckmäßigerweise vorgesehenen Verschlußorgane, die insbesondere aus Streifenförmigen Klettverschlußteilen bestehen, sorgen sehr einfach und schnell für den vor dem Evakuieren notwendigen leichten Anpreßdruck des Kissens an den geschlossenen Schalenkörper. Auf diese Weise läßt sich eine Hülse absolut korrekter Paßform erzeugen und zunächst über den Klettverschluß festlegen. Nach erfolgter Evakuierung liegt dann eine Hülse vor, die in sich verfestigt ist, in ihrer Form stabilisiert ist und die Extremität lagegesichert ist.

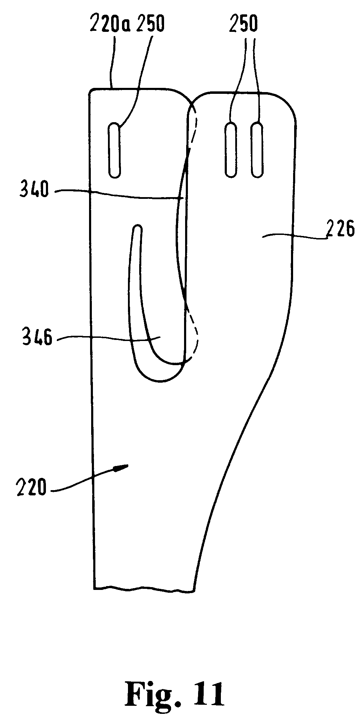

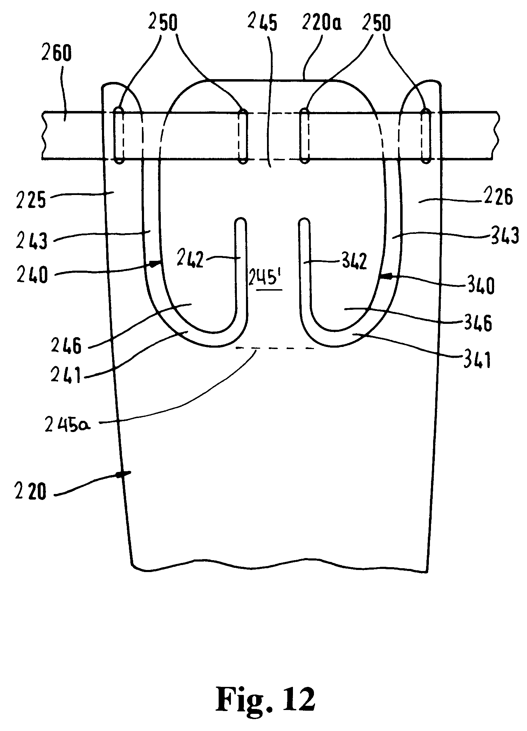

Um den Schalenkörper 210 den jeweiligen vorliegenden Wadengrößen und -formen des Patienten anpassen zu können, ist das rückwärtige Schalenteil 220 mit einer Anzahl von seinem oberen umlaufenden Rand 220a ausgehenden, schlitzförmigen Durchbrechungen 240, 340 versehen, die sich in Schaftlängsrichtung des Schalenteils 220 erstrecken. Bei dem in Fig. 11 und 12 dargestellten Ausführungsbeispiel sind in der rückwärtigen Wand des Schalenteils 220 zwei schlitzförmige Durchbrechungen 240, 340 vorgesehen, die parallel zueinander verlaufend sind und durch die mindestens ein abbiegbarer Abschnitt 245 gebildet wird. Die beiden schlitzförmigen Durchbrechungen 240, 340 erstrecken sich in Schaftlängsrichtung des Schalenteils 220 derart, daß in dem rückwärtigen SChalenteil 220 gegeneinander verschiebbare Wandabschnitte 225, 226, 245 ausgebildet werden, wobei diese Wandabschnitte 225, 226, 245 mit einer Einrichtung 260 auch auf die jeweils erforderliche Stellung fixierbar sein können (Fig. 12). Diese Wandabschnitte 225, 226 sind feststehend und wirken stabilisierend.

Bei dem in Fig. 11 und 12 dargestellten Ausführungsbeispiel sind die beiden schlitzförmigen Durchbrechungen 240, 340 U-förmig verlaufend ausgebildet. Die U-förmigen Durchbrechungsabschnitte sind bei 241, 341 angedeutet. Jede schlitzförmige Durchbrechung 240, 340 geht vom oberen Rand 220a der rückwärtigen Seitenwandung des rückwärtigen Schalenteils 220 aus und verläuft zunächst mit einem Abschnitt 243 in Längsrichtung des Schaftes 221 des Schalenteils 220, wobei dann dieser Abschnitt 243 in den U-förmigen Abschnitt 241 übergeht, dessen auslaufender Schenkelabschnitt 242 in etwa parallel zu dem Durchbrechungsabschnitt 243 verlaufend ist, so wie dies in Fig. 12 dargestellt ist. Die schlitzförmige Durchbrechung 340 ist entsprechend der schlitzförmigen Durchbrechung 240 ausgebildet und wird ebenfalls von einem in Schaftlängsrichtung verlaufenden Abschnitt 343 gebildet, an den sich der U-förmige Abschnitt 341 anschließt, dessen Schenkelabschnitt 342 parallel zu dem Durchbrechungsabschnitt 343 verlaufend ist, so daß die beiden innenliegenden Schenkelabschnitte 242, 342 der beiden Durchbrechungen 240, 340 einander zugekehrt sind. Aufgrund dieser Anordnung und Ausbildung von schlitzförmigen Durchbrechungen 240, 340 in der rückwärtigen Wand des Schalenteils 220 werden lappenförmige Randabschnitte 246, 346 ausgebildet, die die Wirkung von federnd-elastischen Zungen aufweisen und die es möglich machen, daß die die schlitzförmigen Durchbrechungen 240, 340 seitlich begrenzenden Wandabschnitte 225, 226 des rückwärtigen SChalenteils 220 sich gegen die federnden Abschnitte 246, 346 derart verschieben lassen, daß eine Anpassung des Schalenteils 220 und somit des Schalenkörpers 210 an die Wade des Patienten möglich ist. Diese Verschiebbarkeit der federnd-elastischen Wandabschnitte des rückwärtigen Schalenteils 220 ist in Fig. 11 dargestellt.

Die Anordnung der beiden schlitzförmigen Durchbrechungen 240, 340 erfolgt in demjenigen Bereich des rückwärtigen Schalentiels 220 des Schalenkörpers 210, der die Wade des Unterschenkels des Patienten erfaßt.

Der abbiegbare Abschnitt 245 in der Wand des rückwärtigen Schalentiels 220 ist mit dessen Wand über einen Steg 145' verbunden, der federnd-elastisch an der Wand angeformt und gehalten ist oder über eine Gelenkverbindung mit der Wand verbunden ist, so daß im letzteren Fall der Abschnitt 245 die Funktion einer Wippe hat und sich der Wade und der Wadenmuskelbewegungen anpaßt. Im Falle der Verwendung einer Gelenkverbindung erfolgt eine Druckentlastung der Wade, da der Abschnitt 245 quasi freischwingend, doch immer in Anlage an die Wade ist. Vermittels der Gelenkverbindung, die auch als Filmscharnier ausgebildet sein kann, ist der Abschnitt 245 zusammen mit seinem Steg 245' um die Schwenkachse 245a bewegbar (Fig. 12).

Um den rückwärtigen Schalenteil 220 des Schalenkörpers 210 nach erfolgter Anpassung an die Wade des Patienten in der angepaßten Stellung arretieren zu können, weisen die federnden und einzeln ausgebildeten Abschnitte 225, 226, 245 des Schalenteils 220 benachbart zum oberen umlaufenden Rand 220a eine Anzahl von schlitzförmigen Durchbrechungen 250 zur Aufnahme einer streifenförmigen Verschlußeinrichtung 260 auf, die als Gurtband ausgebildet ist oder aus streifenförmigen Klettverschlußteilen bestehen kann, um in der Anpaßstellung das rückwärtige Schalenteil 220 im Bereich seines Schaftes 221 feststellen zu können. Wird der rückwärtige Schalenteil 220 ohne einen vorderen Schalenteil 230 verwendet, dann greifen die Verschlußorgane ausschließlich an den feststehenden Wandabschnitten 225, 226 an. An diesen können die Verschlußorgane auch einendseitig befestigt sein. Diese Art der Anbringung der Verschlußorgane ist jedoch in allen Fällen vorzuziehen, da die freie Beweglichkeit der Abschnitte 245 vermittels dessen Steges 245' nicht beeinträchtigt wird, wobei auch eine Längsverschieblichkeit des Steges nach außen möglich ist.

Die so ausgebildete Fixierungsvorrichtung ist somit mit einer integrierten Wadenanpassung versehen, ohne daß dabei die Formstabilität des Schalenkörpers 210 beeinträchtigt wird. Trotz der hohen Eigensteifigkeit insbesondere des rückwärtigen Schalenteils 220 ist eine Anpassungselastizität an den Unterschenkel und die Wade des Patienten gegeben. Durch den in den rückwärtigen Schalenteil 220 eingesetzten vorderen Schalenteil 230 wird eine Bewegung im rückwärtigen Schalenteil 220 verhindert. Die Normalwinkelstellung zwischen Fuß und Unterschenkel von 90° ist somit immer einhaltbar. Der Unterschenkel liegt bei stationärer Behandlung des Patienten nach einer Operation in dem rückwärtigen Schalenteil 220 und ist in diesem fixiert, so daß die korrekte anatomische Stellung eingehalten werden kann. Trotz der Fixierung des Fußes und des Unterschenkels in der Normalwinkelstellung von 90° wird eine hohe Stabilisierung erreicht.

Um eine Anpassungsfähigkeit des rückwärtigen Schalenteiles 220 an die Wade zu erreichen, kann die Rückwand und/oder die Seitenwände des Schalenteiles 220 auch andersartig ausgebildet sein und verlaufende Einschnitte als wie die vorangehend beschriebenen schlitzförmigen Durchbrechungen 240, 340 aufweisen. Da das Schalenteil 220 aus Kunststoff besteht, ist die Möglichkeit gegeben, anstelle von schlitzartigen Durchbrechungen 240, 340 in Schalenteillängsrichtung verlaufende Abschnitte in Form von Materialverdünnung vorzusehen, die sich ziehharmonikaartig bei einem Verschieben der einzelnen Wandabschnitte gegeneinander verformen.

Die bevorzugterweise aus Kunststoff bestehenden Schalenteile 220, 230 des Schalenkörpers 210 weisen vollwandig ausgebildete Wandflächen auf. Nach einer weiteren Ausführungsform sind die Wandflächen eines jeden Schalenteiles 220, 230 gitterförmig unter Ausbildung von Durchbrechungen zwischen den sich kreuzenden Gitterstäben, die auch streifen- oder bandförmig ausgebildet sein können, ausgebildet. Diese Ausgestaltung erbringt den Vorteil, daß ein zwischen der Schalenkörperwand und der Extremität angeordnetes Manschettenkissen insofern lagegesichert und rutschfest angeordet ist, als die leicht verformbaren Kissenwände in die Durchbrechungen des Schalenkörperwandgitters so geschützt werden, daß Kissenabschnitte wulstartig aus den Durchbrechungen heraustreten und von den die Durchbrechungen begrenzenden Gitterstäben in ihrer Lage gehalten werden.

Des weiteren wirkt der abbiegbare Abschnitt in der Wand des rückwärtigen Schalenteils bei einer Wadenmuskelbewegung als auf die Wade einwirkende Muskelpumpe, wodurch eine Thromboseprophylaxe erreicht wird. Wenn auf das rückwärtige Schalenteil das vordere Schalenteil aufgesetzt ist, dann gibt das vordere Schalenteil den 90°-Winkel für das rückwärtige Schalenteil und somit für die Stellung des Fußes zum Unterschenkel vor. Die seitlichen Wandabschnitte des rückwärtigen Schalenteils sind feststehend und wirken dabei als Stabilisatoren, was besonders dann voreilhaft ist, wenn keine vordere Abdeckung, also kein vorderer Schalenteil, vorgesehen ist. Bei fehlendem vorderen Schalenteil greifen die Verschlußorgane an den feststehenden Wandabschnitten des rückwärtigen Schalenteils an. Das vordere Schalenteil dient als Stabilisator und gibt den 90°-Winkel vor.

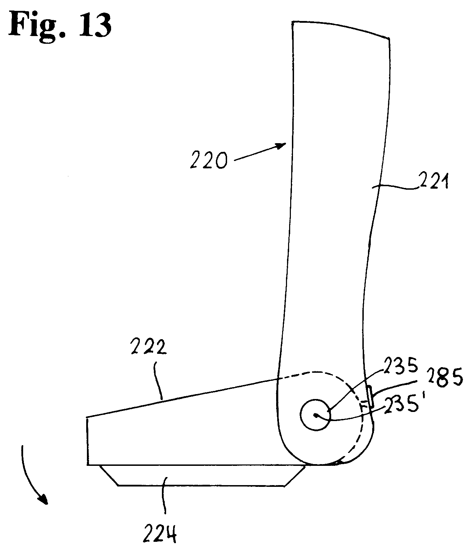

Die Vorrichtung zur umschließenden Fixierung von Extremitäten besteht gemäß Fig. 13 ebenfalls aus einem rückwärtigen Schalenteil 220, das eine L-Form aufweist und aus einem Schaft 221 und einem Fußteil 222 besteht, wobei das Fußteil 222 über seitliche Schwenkgelenke 235 um eine Schwenkachse 235a verschwenkbar ist, so daß der Winkel des Fußteils 222 zu dem Schaft 221 einstellbar ist. Dieses Schalenteil 220 besteht zweckmkäßigerweise aus Kunststoff.

Das Fußteil 222 und der Schaft 221 des rückwärtigen Schalenteiles 220 sind schalenförmig nach oben und nach vorn offen ausgebildet, so daß eine Aufnahmewanne für den Fuß und den Unterschenkel geschaffen ist. Die weitere Ausgestaltung entspricht den vorherigen Ausführungen zum rückwärtigen Schalenteil 220.

Auch hier wird vor dem Einlegen der Extremität in das rückwärtige Schalenteil 220 zwischen dieser und dem Schalenteil ein verformbares, vakuumdicht ausgebildetes und mit zumindest einem Ventil versehenes Kissen 20 eingelegt.

Sollte jedoch eine, die Extremität umschließende Fixierung gewünscht oder erforderlich werden, kann das rückwärtige Schalenteil 220 mit einem vorderen Schalenteil 230 zu einem Schalenkörper 210 zusammengesetzt werden, wobei auch das vordere Schalenteil 230 eine L-Form mit einem Schaft 231 und einem Fußteil 232 aufweist. Das Fußteil 232 kann zum Schaft 232 des vorderen Schalenteiles 230 feststehend und in einer vorgegebenen Winkelstellung z. B. von 90° angeordnet sein. Ein derart vorderes Schalenteil 230 wird immer dann zur Anwendung gelangen, wenn die Extremität im rückwärtigen Schalenteil 220 bei einer Winkelstellung von 90° des Fußes zum Unterschenkel des Patienten angeordnet ist. Es besteht auch die Möglichkeit, ein vorderes Schalenteil 230 einzusetzen, bei dem das Fußteil 232 zum Schaft 231 in verschiedenen Winkelstellungen angeordnet werden kann, wobei dann das Fußteil 232 in der vorgegebenen Winkelstellung arretierbar ist.

Auch das vordere Schalenteil 230 ist in Richtung zum Fuß und zum Unterschenkel schalenförmig ausgebildet, wobei dan beide Schalenteile 220, 230 so ausgebildet sind, daß das vordere Schalenteil 230 in das rückwärtige Schalenteil 220 derart einsetzbar ist, daß die seitlichen Wandabschnitte des Schalenteiles 220 die seitlichen Wandabschnitte des Schalenteiles 230 abschnittsweise übergreifen.

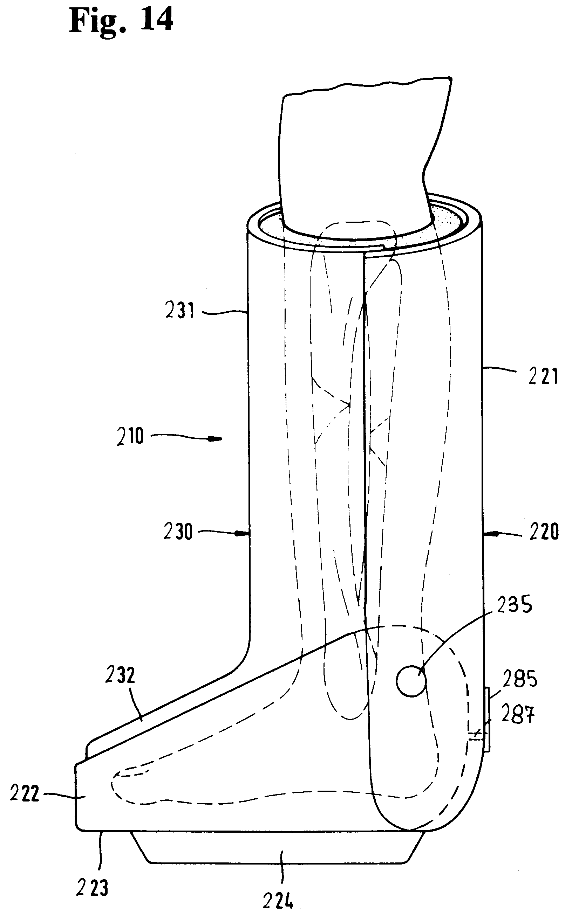

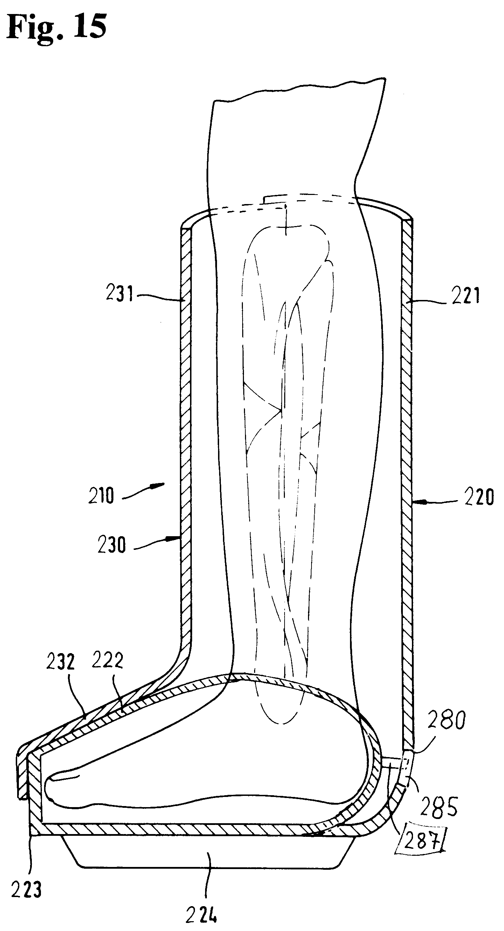

Um eine wahlweise Winkelverstellung des Fußes zum Unterschenkel bzw. des Fußteils 222 zum 221 des rückwärtigen Schalenteiles 220 vornehmen zu können, ist in der rückwärtigen Wandfläche des Schaftes 221 des Schalenteiles 220, und zwar in dem den Fußteil 222 gegenüberliegenden Wandbereich eine fensterartige Durchbrechung 280 ausgebildet, in die ein plattenförmiger Winkeladapter 285 einsetzbar ist, der mit einer Eingrifföffnung 286 versehen ist, in die zur Winkelfestlegung zwischen dem Fußteil 222 und dem Schaft 221 des rückwärtigen Schalenteiles 220 ein Nocken 287 eingreift, der an dem Fußteil 222 des rückwärtigen Schalenteiles 220 angeformt ist (Fig. 14, 15 und 17).

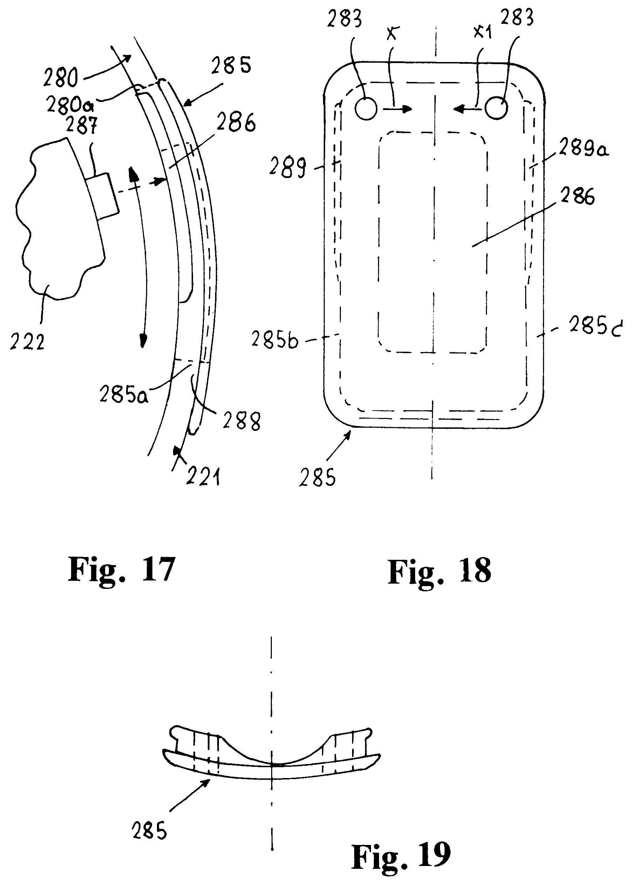

Die in diese fensterartige Durchbrechung 280 in der Rückwand des rückwärtigen Schalenteiles 220 einsetzbaren Winkeladapter 285 weisen Eingriffsöffnungen 286 für die Nocken 287 in unterschiedlichen Lagenanordnungen zur Winkeladapterfläche auf, um den Fußteil 222 zum Schaft 221 des rückwärtigen Schalenteiles 220 in vorgegebenen und bestimmten Winkelstellungen fixieren zu können (Fig. 18 und 19).

Die fensterartige Durchbrechung 280 in der Wand des rückwärtigen Schalenteiles 220 ist etwa rechteckförmig ausgebildet. Der Winkeladapter 285 weist eine der Form und den Abmessungen dieser fensterartigen Durchbrechung 280 entsprechende Ausgestaltung auf, um beispielsweise mittels Klemmsitz in der fensterartigen Durchbrechung 280 gehalten zu werden. Die Länge der Eingrifföffnung 286 in dem Winkeladapter 285 entspricht nach einer Ausführungsform einem einstellbaren Winkelbereich des Fußteils 222 zu dem Schaft 221 des Schalenteiles 220.

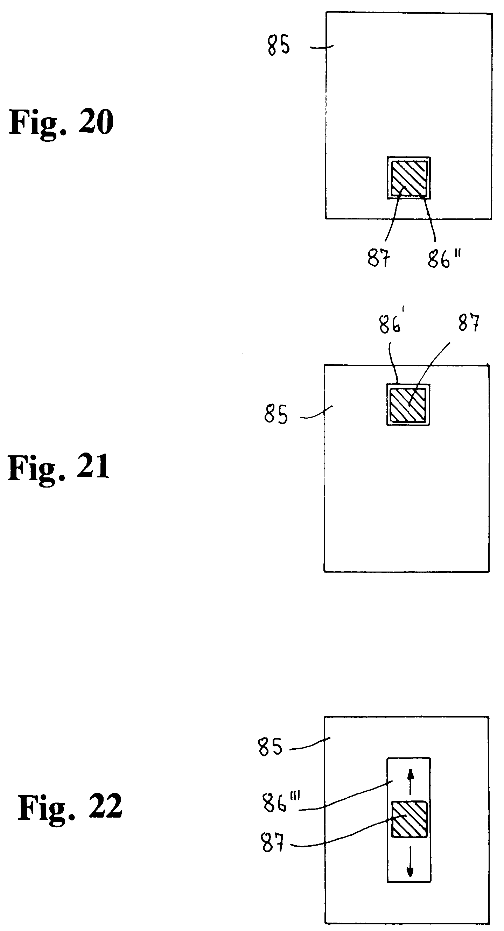

Um Winkeladapter 285 für die verschiedensten Winkelbereiche des Fußteils 222 zu dem Schaft 221 des Schalenteiles 220 zur Verfügung zu haben, ist nach einer Ausführungsform die Eingrifföffnung 286'' für den Nocken 287 im unteren Bereich des Winkeladapters 285 ausgebildet (Fig. 20). Bei dieser Ausführungsform wird eine Winkelstellung des Fußteils 222 zu dem Schaft 221 des Schalenteils 220 in einem Winkel von 90° erhalten, was der Normalwinkelstellung zwischen dem Fuß und dem Unterschenkel entspricht.

Soll eine Spitzfußstellung von 120° als Winkel zwischen dem Fußteil 222 und dem Schaft 221 erhalten werden, dann findet ein Winkeladapter 285 Verwendung, bei dem die Eingrifföffnung 286' für die Nocken 287 im oberen Bereich des Winkeladapters 285 ausgebildet ist (Fig. 21).

Um in einem größeren Winkelbereich variabel sein zu können, kann der Winkeladapter 285 auch eine sich über die Adapterlänge erstreckende, zweckmäßigerweise schlitzförmige Eingrifföffnung 286''' für den Nocken 287 aufweisen, wobei es jedoch bei dieser Ausführungsform dann vorteilhaft ist, wenn der Nocken 287 beispielsweise als Feststellschraube ausgebildet ist (Fig. 22).

Werden z. B. drei Winkeladapter 285 mit verschiedenen Anordnungen der Eingrifföffnungen 286', 286'' und 286''' zur Verfügung gestellt, so ist mit diesen drei Winkeladaptern 285 die gesamte Palette von Winkelstellungen erhältlich und einstellbar, wobei auch der Winkeladapter 285 mit den Eingrifföffnungen 286' und 286'' als ein einziges Bauelement eingesetzt werdenkann, wobei dann dieser Winkeladapter 285 entweder im oberen Bereich oder in seinem unteren Bereich eine Eignrifföffnung 286' bzw. 286'' aufweist, wodurch die Möglichkeit gegeben ist, durch unterschiedliches Einsetzen dieses Winkeladapters in die fensterartige Durchbrechung 280 beide Winkelbereiche wahlweise zu erfassen, nämlich einmal den Bereich mit dem Winkel von 90° und dem Winkel von 120°, so daß lediglich der in Fig. 20 dargestellte Winkeladapter 285 um 180° verdreht dann in die fenterartige Durchbrechung 280 in dem Schaft 221 des rückwärtigen Schalenteiles 220 eingesetzt wird, um die Eingrifföffnung 286' im oberen Bereich zu erhalten, so daß dann bei einem Eingriff des Nockens 287 in die diese Lage einnehmende Eingrifföffnung 286' die Spitzfußstellung erreicht und erhalten wird, so daß allein schon mit einem einzigen Winkeladapter die Winkelstellungen von 90° und 120° erreichbar sind. Hierbei ist jedoch die Anordnung und Zuordnung der Eingrifföffnung in dem Winkeladapter 285 zur gesamten Fläche des Winkeladapters derart, daß bei einem Einsetzen des Winkeladapters 285 nach erfolgter Drehung um 180° auch der für die Spitzfußstellung erforderliche Winkel erreicht wird.

Der plattenförmige Winkeladapter 285 ist beispielsweise vermittels Klemmsitz in der fensterartigen Durchbrechung 280 im Schaft des rückwärtigen Schalenteils 220 gehalten. Entsprechend Fig. 17 bis 19 ist der plattenförmige Winkeladapter 285 an seinem umlaufenden Rand 285a mit einer umlaufenden oder sich über sich gegenüberliegende Bereiche erstreckende Nuten 288 zum Eingriff des die fenterartige Durchbrechung 280 begrenzenden Randes 280a versehen. Bevorzugterweise besteht der plattenförmige Winkeladapter 285 aus einem federnd-elastischen Material, zweckmäßigerweise aus einem Kunststoff, und ist dabei mittels Klemmsitz am umlaufenden Rand 280a der fensterartigen Durchbrechung 280 gehalten. Aufgrund der federnd-elastischen Ausgestaltung der Randabschnitte wird der plattenförmige Winkeladapter 285 lediglich in die fensterartige Durchbrechung 280 derart eingedrückt, daß der Winkeladapter 285 im Randbereich der fensterartigen Durchbrechung 280 gehalten ist. Der umlaufende Rand des Winkeladapters 285 und der die fensterartige Durchbrechung 280 begrenzende Rand ist mit Eingriffprofilierungen versehen, d. h. nach Art der Nut- und Feder-Ausgestaltung kann eine Randprofilierung vorgenommen werden.

Nach einer weiteren Ausführungsform gemäß Fig. 18 weist der plattenförmige Winkeladapter 285 an seinen beiden Längsseitenkanten 285b, 285c keilförmig sich nach oben erweiternde Verstärkungen 289, 289a aus einem federndelastischen Material auf, so daß der Klemmsitz im Randbereich der fensterartigen Durchbrechung 280 noch erhöht wird. Diese sich keilförmig nach oben erweiternden Verstärkungen 289, 289a an den seitlichen Rändern des plattenförmigen Winkeladapters 285 können auch als bewegbare Leisten ausgebildet sein, die mit einem Mechanismus derart verbunden sind, daß bei einer Betätigung des Mechanismus diese keilförmigen Verstärkungen 289, 289a in den Rand des Winkeladapters 285 eingeschwenkt werden, so daß, wenn der Winkeladapter 285 nicht mittels Klemmsitz in der fensterartigen Durchbrechung 280 gehalten ist, dann nach dieser sogenannten Entriegelung der Winkeladapter 285 aus der fensterartigen Durchbrechung 280 herausgenommen werden kann. Auch andersartig ausgebildete Verriegelungseinrichtungen für die Arretierung und Halterung des Winkeladapters 285 in der fensterartigen Durchbrechung 280 können vorgesehen sein.

Das Auswechseln des Winkeladapters 285 kann mittels in der Zeichnung nicht dargestellter zangenartiger Öffnungswerkzeuge erfolgen. Hierzu weist der plattenförmige Winkeladapter 285 in seinem oberen Bereich zwei im Abstand voneinander angeordnete Durchbrechungen 283 auf, in die ein zangenartiges Öffnungswerkzeug einsetzbar ist, so daß bei aufeinanderlaufenden Bewegungsrichtungen in Pfeilrichtung X, X1 der Winkeladapter 285 in diesem Eingriffbereich des Werkzeuges zusammengepreßt wird und somit die Entriegelung der leistenartigen Verstärkungen 289, 289a aus dem Randbereich der fensterartigen Durchbrechung 280 erfolgt. vorausgesetzt ist, daß eine entsprechende federnd-elastische Ausgestaltung des Winkeladapters 285 gegeben ist, wobei jedoch der Winkeladapter 285 im Bereich der Eingrifföffnung 286 für den Nocken 287 eine hohe Eigensteifigkeit aufweisen muß.

Die Eingrifföffnungen 286, 286', 286'', 286''' in dem Winkeladapter 85 für den Nocken 287 brauchen nicht als Durchbrechung ausgebildet zu sein; auch nutenförmige Vertiefungen oder Ausnehmungen können in dem Winkeladapter 285 die Lagesicherung und Fixierung des Nockens 287 vornehmen.

Es besteht jedoch auch die Möglichkeit, den Winkeladapter 285 im rückwärtigen Bereich des Fußteils 222 des rückwärtigen Schalenteils 220 anzuordnen, während der Nocken 287 oder ein entsprechend andersartig ausgebildetes Eingriffelement an der Innenwandfläche der Rückwand des Schalenteiles 220 anzuordnen ist.

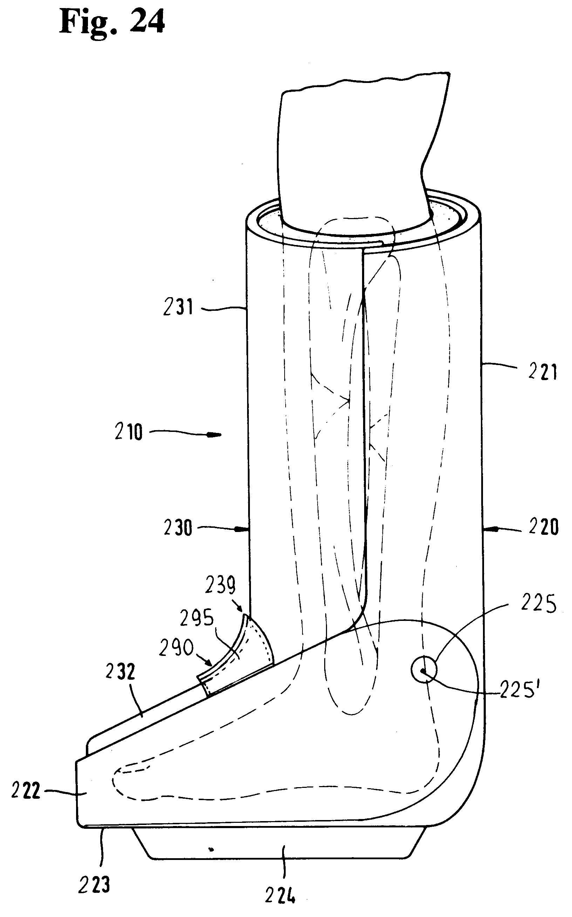

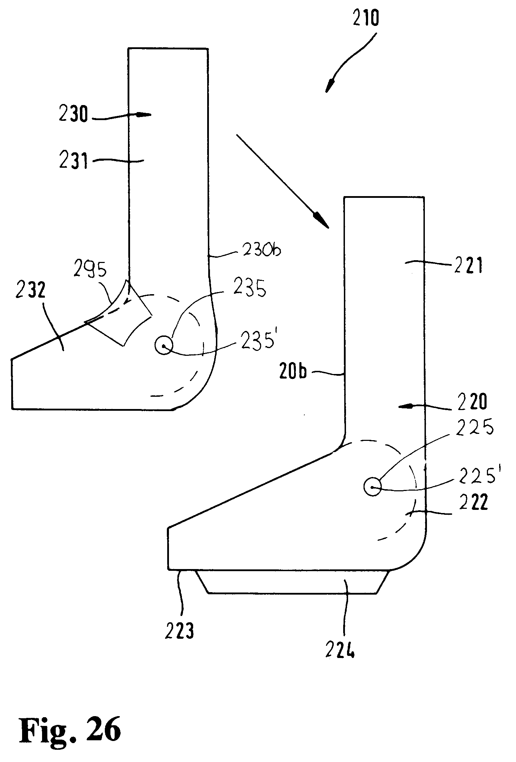

Eine Vorrichtung zur umschließenden Fixierung von Extremitäten ist ferner in Fig. 24, 25 und 26 als Schalenkörper 210 für den Fuß-/Unterschenkel-Bereich eines Patienten dargestellt. Der Schalenkörper 210 ist auch bei diesem Ausführungsbeispiel entsprechend dem vorhergehend beschriebenen Schalenkörper 210 ausgebildet.

Die Fußteile 222, 232 der beiden Schalenteile sind mit ihren Schäften 221, 231 über seitliche Schwenkgelenke 225, 235 miteinander verbunden, so daß die Fußteile 222, 232 zu den schäften 221, 231 um Schwenkachsen 225a, 235a verschwenkbar sind. Sowohl die beiden Schalenteile 229, 230 als auch ihre Schwenkgelenke 225, 235 bestehen zweckmäßigerweise aus Kunststoff.

Zwischen den beiden gegeneinander verspannbaren Schalenteilen 220, 230 und der Extremität ist ebenfalls ein Manschettenkissen 20 angeordnet.

Eine Bewegung im Fußgelenk des Patienten kann verhindert werden, indem der Neigungswinkel des Schaftteils bezüglich des Fußteils um die Schwenkachse 225 bzw. 235 fest eingestellt wird. eine Wahl dieses fest eingestellten Winkels kann in Abhängigkeit von der zu behandelnden Verletzung innerhalb des gesamten einstellbaren Schwenkwinkelbereiches erfolgen. Das Feststellen auf einen bestimmten Winkel erfolgt mittels entsprechend ausgebildeter Verstelleinrichtungen, wobei bei dem rückwärtigen Schalenteil 220 eine Winkelvorgabe zur Einstellung eines Winkels von 90° oder von 120° zwischen dem Fußteil 222 und dem Schaft 221 vermittels eines in der Zeichnung nicht dargestellten Winkeladapters erfolgen kann.

Um eine Anpassung des vorderen Schalenteiles 230 an die Winkelstellung des Fußteiles 222 zum Schaft 221 des rückwärtigen Schalenteils 220 vornehmen zu können, ist das vordere Schalenteil 230 mit einer Winkeleinstelleinrichtung 290 versehen, vermittels der das Fußteil 232 zum Schaft 231 in einer vorgegebenen Winkelstellung arretierbar ist, wobei diese Winkelstellung mit derjenigen Winkelstellung des Fußteils 222 zum Schaft 221 des rückwärtigen Schalenteils 220 übereinstimmt.

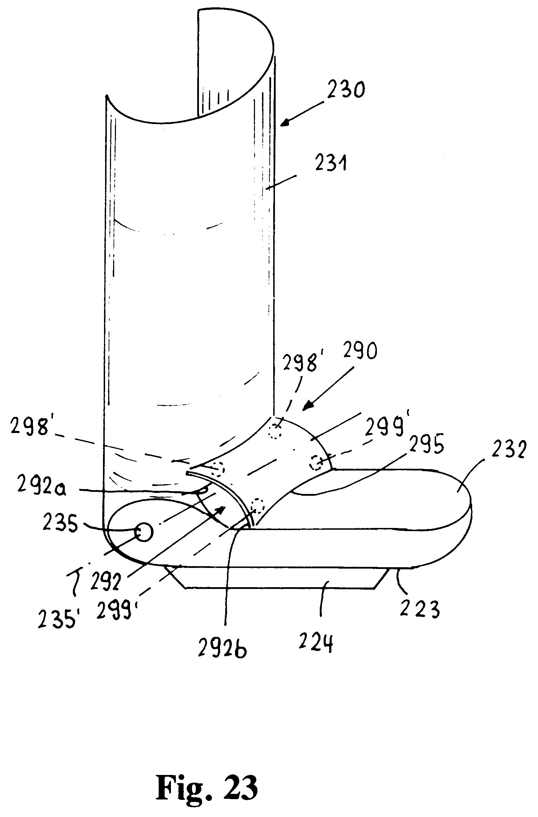

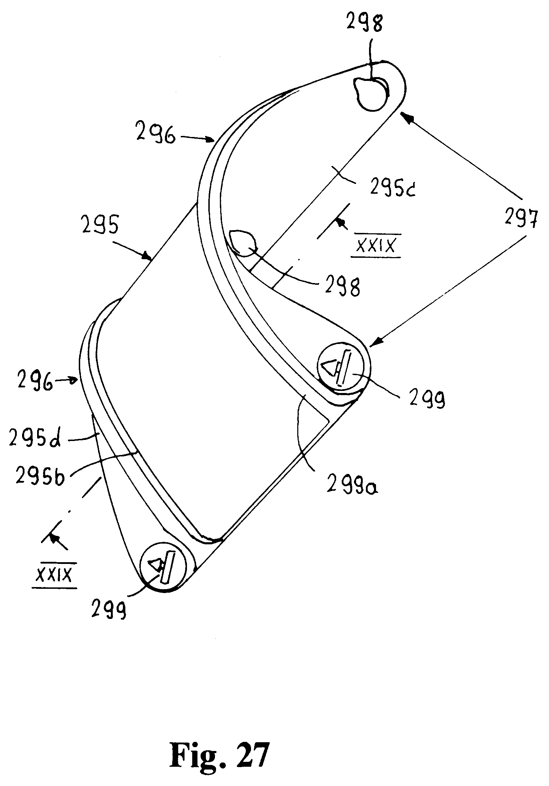

Diese Winkelstelleinrichtung 290 besteht aus einem zwischen dem Fußteil 232 und dem Schaft 231 des vorderen Schalenkörpers 230 angeordneten, dem Außenprofil des Schalenkörpers 210 bzw. des vorderen Schalenteils 230 entsprechend geformten, plattenförmigen Winkeladapter 295, der mittels Rast- und Verriegelungseinrichtungen 297 an dem Schaft 231 und dem Fußteil 232 des vorderen Schalenteils 230 lösbar gehalten ist (Fig. 23 und 27). dieser Winkeladapter 295 übergreift laschenförmig vorderseitig den Verbindungsbereich 239 zwischen dem Fußteil 232 und dem Schaft 231 des vorderen Schalenkörpers 230.

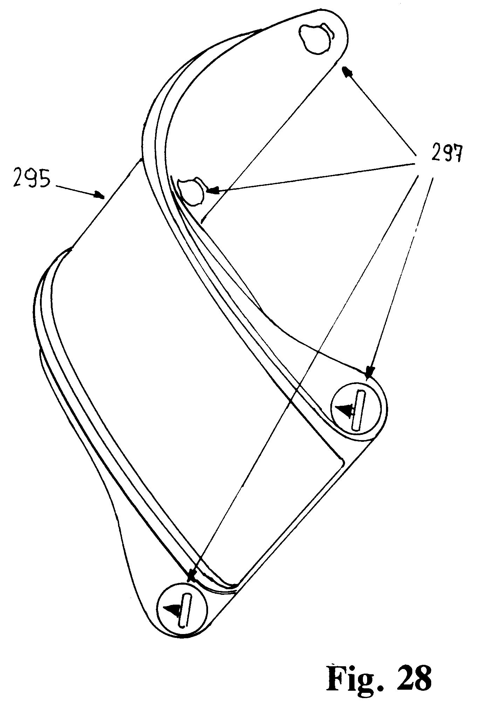

Dieser plattenförmige Winkeladapter 235 weist eine Breite auf, aufgrund der das Fußteil 232 zu dem Schaft 231 des Schalenteils 230 eine Winkelstellung von 90° einnimmt (Fig. 27). Bei dieser Winkelstellung handelt es sich um die Normalstelung zwischen dem Fuß und Unterschenkel. Eine weitere Ausführungsform des Winkeladapters 295 sieht eine Breite vor, aufgrund der das Fußteil 232 zu dem Schaft 231 des Schalenteils 230 eine Winkelstellung von 120° einnimmt, so daß, wenn diese Ausführungsform des Winkeladapters zwischen dem Fußteil 232 und dem Schaft 231 des Schalenteils 230 eingesetzt wird, das Fußteil zu dem Schaft 231 die Spitzfußwinkelstellung erhalten wird (Fig. 28).

Das Fußteil 232 und der Schaft 231 des vorderen Schalenteils 230 weisen im Verbindungsbereich 239 dieser beiden Teile eine fensterartige Durchbrechung 292 auf, die sich bis etwa in den Seitenbereich des Fußteiles 232 und des Schaftes 231 erstreckt, so daß der an dem Schalenteil 230 angebrachte Winkeladapter 295 diese fensterartige Durchbrechung 292 in Form einer Abdecklasche übergreift, wobei die Abmessungen des Winkeladapers 295 derart bemessen sind, daß dieser in die fensterartige Durchbrechung 232 einsetzbar bzw. mit seinem umlaufenden Rand auf den die fensterartige Durchbrechung 292 begrenzenden Rand aufsetzbar und hier mittels Klemm- bzw. Preßsitz gehalten ist.

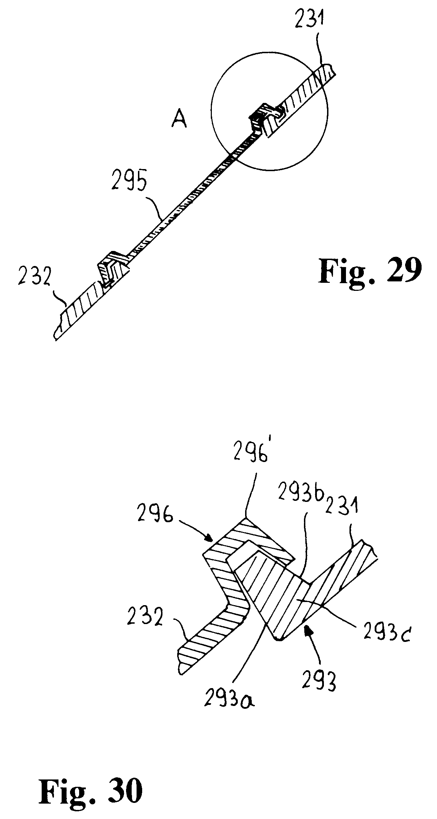

Zur Halterung des Winkeladapters 295 an den Wandflächen des Fußteiles 232 und des Schaftes 231 des Schalenteils 230 weisen die gegenüberliegenden, quer zur Schaftlängsrichtung verlaufenden Ränder 292a, 292b der fensterartigen Durchbrechung 292 außenseitig wulstartig oder andersartig profilierte leistenförmige Verstärkungen 293 auf, während der Winkeladapter 295 an seinem oberen Rand 295a und an seinem unteren Rand 295b mit den Verstärkungen 293 an den Rändern 292a, 292b entsprechend ausgebildeten Gegenprofilen als Randprofile 296 versehen sind, so daß der Winkeladapter 295 mittels Klemmsitz an den Verstärkungen 293 an dem Fußteil 232 oder dem Schaft 231 bzw. an diesem des vorderen Schalenteils 230 gehalten ist (Fig. 29).

Die Randverstärkungen 293 an dem Fußteil 232 und dem Schaft 231 des vorderen Schalenteils 230 weisen ein Querschnittsprofil mit einer quadratischen oder rechteckförmigen, konisch nach oben verlaufende Seitenflächen 293a, 293b aufweisenden Fläche 293c auf (Fig. 30). Die Randprofile 296 an dem plattenförmigen Winkeladapter 295 sind als entsprechendes, die Randverstärkung 293 an dem Fußteil 232 und dem Schaft 231 übergreifendes Gegenprofil 296' ausgebildet.

Die Befestigung und Halterung des Winkeladapters 295 an dem Schaft 231 und dem Fußteil 232 des vorderen Schalenteils 30 erfolgt entsprechend Fig. 27 vermittels zweier an dem einen Ende 295c des Winkeladapters, und zwar innenwandseitig feststehend angeordneten Verriegelungshaken 298, die in Durchbrechungen 298' in den Wandflächen des Schaftes 231 und des Fußteiles 232 des Schalenteiles 230 eingreifen. Mit diesem seinem Ende 295c wird der Winkeladapter 295 in die vorgesehenen Durchbrechungen 298' in den Wandflächen des Schaftes 231 und des Fußteiles 232 des Schalenteils 230 eingehakt. An seinem anderen Ende 295d trägt der Winkeladapter 295 zwei drehbare, als Exzenter ausgebildete Verriegelungshaken 299, die ebenfalls in entsprechende Durchbrechungen 299' in den Wandflächen des Schaftes 231 und des Fußteiles 232 des Schalenteils 230 eingreifen, wobei die Verriegelung durch Verdrehen der in diese Durchbrechungen 299' eingeführten Verriegelungshaken 299 erfolgt, so daß durch diese drehbaren und als Exzenter ausgebildeten Verriegelungshaken 299 der Winkeladapter 295 über die Gegenteile, hier Fußteil 232 und Schaft 231, gespannt wird, so daß dadurch ein spielfreier Preßsitz, insbesondere an den schrägen Flächen der wulstartigen Randvrstärkungen 193 erreicht wird (Fig. 30). Die Anzahl der feststehenden Verriegelungshaken 298 und der verdrehbaren Verriegelungshaken 299 kann beliebig gewählt sein. Wesentlich ist, daß jeweils ein feststehender Verriegelungshaken 298 und ein verdrehbarer Verriegelungshaken 299 miteinander korrespondieren und in die Durchbrechungen 298' im Schaft 231 und in die Durchbrechungen 299' im Fußteil 232 des Schalenteils 230 eingreifen.

Anstelle von Verriegelungshaken 298, 299 können jedoch auch andersartig ausgestaltete Befestigungsmittel eingesetzt werden.

Der plattenförmige Winkeladapter 295 besteht bevorzugterweise aus einem federnd-elastischen Material, zweckmäßigerweise aus einem Kunststoff.

Auch die beiden Schalenteile 220, 230 bestehen aus einem Kunststoff.

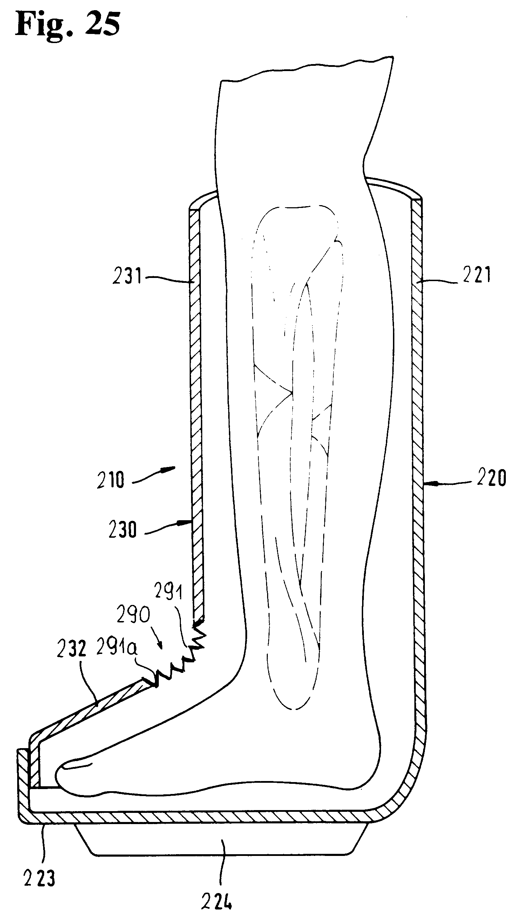

Eine weitere Möglichkeit der Winkelverstellbarkeit des Fußteiles 232 zum Schaft 231 des vorderen Schalenteils 230 besteht gemäß Fig. 25 darin, daß das zum Schaft 231 verstellbare Fußteil 232 des vorderen Schalenteiles 230 über einen quer zur Schaftlängsrichtung verlaufenden, zieharmonikabalgartig ausgebildeten Abschnitt 291 mit einer Anzahl von gefalteten und filmscharnierartig miteinander verbundenen Materialstreifen 291a verbunden ist, wobei die das Fußteil 232 und den Schaft 231 miteinander verbindenden, seitlich an dem Schalenkörper 210 ausgebildeten Schwenkgelenke 235 mit Winkelarretierungseinrichtungen verehen sind. Bei dieser Ausführungsform ist der der sonst mittels des plattenförmigen Winkeladapters 295 verschlossenen fensterartigen Durchbrechung 292 zwischen dem Fußteil 232 und dem Schaft 231 entsprechende Bereich über einen Abschnitt 291 ausgefüllt, der aus dem gleichen Material besteht, aus dem auch der Schalenteil 230 gefertigt ist. Dieser Abschnitt 291 ist balgartig entsprechend einer Ziehharmonika ausgebildet, wobei die einzelnen Materialstreifen 219a filmscharnierartig verbunden sind, so daß in Abhängigkeit von der jeweiligen Winkelstellung des Fußteiles 232 zu dem Schaft 231 des chalenteils 230 sich dieser dehnbare Abschnitt 291 anpaßt. Die im Bereich der Schwenkgelenke 235 vorgesehenen Winkelarretierungseinrichtungen sind in an sich bekannter Weise ausgebildet; sie bestehen aus Feststelleinrichtungen, z. B. Feststellschrauben od. dgl., so daß die vorgegebene Winkelstellung beibehalten und nur nach Lösen der Winkelarretierungseinrichtung verändert werden kann.

Die Fixierungsvorrichtung für Extremitätenfrakturen im Bereich von Unterschenkel und Oberschenkel kann mit gleich gutem Ergebnis auch bei Extremitätenfrakturen im Bereich von Oberarm und Unterarm eingesetzt werden.

## Patentansprüche

1. Vorrichtung zur umschließenden Fixierung von Extremitäten und Extremitätenbereichen, insbesondere zur Behandlung von Extremitätenfrakturen im Bereich von Unterschenkel und Oberschenkel, bestehend aus einem Schalenkörper (210) aus einem Schalenteil (220) oder aus zwei gegeneinander verspannbaren Schalenteilen (220, 230), die die zu umschließende Extremität umgeben oder aufnehmen, wobei zwischen den Schalenteilen (220, 230) und der Extremität mindestens ein evakuierbares Manschettenkissen (20) vorgesehen ist, in dessen zwischen den beiden Kissenwänden gelegenem Innenraum eine Vielzahl von relativ zueinander beweglichen Füllkörpern vorgesehen ist, wobei das Manschettenkissen (20) eine Mehrzahl von untereinander verbundenen und mit Füllkörpern gefüllten vakuumdichten Bereichen (400) aufweist, zwischen denen Stege (410) und Inseln (420) mit Luftdurchtrittsöffnungen (411,421) ausgebildet sind,
dadurch gekennzeichnet,
daß das Manschettenkissen (20) im Bereich eines Muskelpolsters der zu umschließenden Extremität, vorzugsweise im Bereich der Wade eines zu umschließenden Unterschenkels, einen ersten Inselbereich (430), der mit Luftdurchtrittsöffnungen (431) versehen ist und an den seitlich und unten vakuumdichte Bereiche (400) des Kissens (20) angrenzen, und im Bereich des Fersenaufstandspunktes einen zweiten Inselbereich (440) aufweist, der mit Luftdurchtrittsöffnungen (441) versehen ist und der von vakuumdichten Bereichen (400) des Kissens (20) vorzugsweise allseitig umgeben ist.

2. Vorrichtung nach Anspruch 1,
dadurch gekennzeichnet,
daß im Fußbereich und/oder im Bereich der Achillessehne eine saugfähige Einlage (20') vorgesehen ist, die an ihrer zur Extremität (E) weisenden Seite eine für Dampf und/oder Feuchtigkeit semipermeable Schicht (20'') aufweist.

3. Vorrichtung nach einem der Ansprüche 1 und 2, bei der im Vußteil Lüftungsdurchlässe vorgesehen sind,
dadurch gekennzeichnet,
daß ein erster Schalenteil (11) einen Fußteil (10) umfaßt, in dem ein Fußbett (51) angeordnet ist, das vertikale Lüftungsdurchlässe (51') aufweist, die in Längskanäle (51'') und/oder Querkanäle (51''') münden, welche an der Unterseite des Fußbettes (51) vorgesehen sind.

4. Vorrichtung nach Anspruch 3,
dadurch gekennzeichnet,
daß die Querkanäle (51''') an den Außenseiten des Fußbetts entlang im wesentlichen nach oben verlaufen und sich vorzugsweise nach oben öffnen.

5. Vorrichtung nach einem der Ansprüche 3 und 4,
dadurch gekennzeichnet,
daß die Querkanäle (51''') mit im Fußteil (10) gelegenen Belüftungsöffnungen (70) ausgerichtet sind.

6. Vorrichtung nach einem der Ansprüche 1 bis 5,
dadurch gekennzeichnet,
daß ein erster Schalenteil (11) einen Fußteil (10) umfaßt, in dem eine Zwischensohle (50) angeordnet ist, die an ihrer Vorderseite im vorderen Abschnitt des Fußteils (10) um eine Querachse schwenkbar gelagert ist und die in ihrem rückwärtigen fersenseitigen Endbereich von einem Spannelement (72), vorzugsweise einem Spannband, untergriffen ist, wobei das Spannband im Bereich des Übergangs vom Fußteil zum Schaftteil verläuft und außen über der die freien Ränder von Schaft- und Fußteil übergreifenden Schaftabdeckschale (12) zusammenspannbar ist.

7. Vorrichtung nach Anspruch 6,
dadurch gekennzeichnet,
daß außen am ersten Schalenteil (11) im Bereich des Übergangs zum Fußteil Verrastungseinrichtungen (11') vorgesehen sind, die mit zugeordneten, innen an der Schaftabdeckschale (12) vorgesehenen Verrastungseinrichtungen (12') derart zusammenwirken, daß ein Festspannen der Spanneinrichtung (72) ein gegenseitiges Verrasten der Verrastungseinrichtung (11', 12') bewirkt.

8. Vorrichtung nach einem der Ansprüche 1 bis 7,
dadurch gekennzeichnet,
daß an der Innenseite der Schaftabdeckschale (11) Verhakungseinrichtungen für ein Kissen (20), vorzugsweise im wesentlichen horizontal verlaufende Stege (11''), ausgebildet sind.

9. Vorrichtung nach einem der Ansprüche 1 bis 8,
dadurch gekennzeichnet,
daß die beiden Schalenteile aus einem Kunststoff bestehen.

10. Vorrichtung nach einem der Ansprüche 1 bis 9,
dadurch gekennzeichnet,
daß die Wandflächen eines jeden Schalenteiles (220; 230) vollwandig ausgebildet sind.

11. Vorrichtung nach einem der Ansprüche 1 bis 10,
dadurch gekennzeichnet,
daß die Wandflächen eines jeden Schalenteiles (220; 230) gitterförmig ausgebildet sind.

12. Vorrichtung nach einem der Ansprüche 1 bis 11,
dadurch gekennzeichnet,
daß von den beiden, eine etwa L-Form aufweisenden Schalenteilen (220, 230) mit einem etwa halbkreisförmigen Querschnittsprofil das rückwärtige Schalenteil (220) unter Ausbildung mindestens eines abbiegbaren Abschnittes (245) vom oberen Schalenteilenrand (220a) ausgehend schlitzförmige Durchbrechungen (240, 340) aufweist, die sich über einen Bereich in Schalenteillängsrichtung derart erstrecken, daß in dem rückwärtigen Schalenteil (220) federnd-elastische und gegeneinander verschiebbare Wandabschnitte (225, 226, 245) ausgebildet sind, und daß eine diese Abschnitte (225, 226, 245) fixierende Einrichtung (260) vorgesehen ist.

13. Vorrichtung nach einem der Ansprüche 1 bis 12,
dadurch gekennzeichnet,
daß von den beiden eine etwa L-Form aufweisenden Schalenteilen (220; 230) mit einem etwa halbkreisförmigen Querschnittsprofil und mit einem Schaft (221; 231) und einem Fußteil (222; 232) das rückwärtige Schalenteil (220) ein zu dessen Schaft (221) winkelverstellbares Fußteil (222) und in dem dem Fußteil (222) gegenüberliegenden Wandbereich eine fensterartige Durchbrechung (80) mit einem in diese eingesetzten, austauschbaren plattenförmigen Winkeladapter (285) aufweist, der mit einer Eingriffsvertiefung oder Eingrifföffnung (286) versehen ist, in die zur Winkelfestlegung zwischen dem Fußteil (222) und dem Schaft (221) des rückwärtigen Schalenteiles (220) ein Nocken (287) eingreift, der zur Fixierung einer vorbestimmten Winkelstellung in dem rückwärtigen Bereich des Fußteils (22) an diesem angeformt ist.

14. Vorrichtung nach einem der Ansprüche 1 bis 13,
dadurch gekennzeichnet,
daß von den beiden eine etwa L-Form aufweisenden Schalenteilen (220, 230) mit einem etwa halbkreisförmigen Querschnittsprofil und mit einem Schaft (221, 231) und einem Fußteil (222, 232), das vordere Schalenteil (230) ein zu dessen Schaft (231) winkelverstellbares Fußteil (232) und in dem Bereich zwischen dem Fußteil (232) und dem Schaft (231) eine Winkeleinstelleinrichtung (290) aufweist, vermittels der das Fußteil (232) zum Schaft (231) in einer vorgegebenen Winkelstellung arretierbar ist, wobei das rückwärtige Schalenteil (220) ein zu dessen Schaft (221) winkelverstellbares Fußteil (222) oder ein zu dessen Schaft (221) in einem vorgegebenen Winkel festgestelltes Fußteil (222) aufweist.

15. Vorrichtung nach Anspruch 12,
dadurch gekennzeichnet,
daß in dem rückwärtigen Schalenteil (220) zwei im Abstand, in Schalenteillängsrichtung verlaufende und sich vom oberen Schalenrand (22a) erstreckende schlitzförmige Durchbrechungen (240, 340) ausgebildet sind, die in etwa U-förmige Abschnitte (241, 341) mit parallel zueinander verlaufenden schlitzförmigen Schenkelabschnitten (242, 342) auslaufen, so daß in der Wand des rückwärtigen Schalenteils (220) zwei zungenförmige Wandabschnitte (246, 346) zu den schlitzförmigen Durchbrechungen (240, 340) benachbarten Wandabschnitte (225, 226) ausgebildet sind, wobei die zu den schlitzförmigen Durchbrechungen (240, 340) benachbarten Wandabschnitte (225, 226) feststehend sind.

16. Vorrichtung nach einem der Ansprüche 12 und 15,
dadurch gekennzeichnet,
daß der zwischen den schlitzförmigen Schenkelabschnitten (242, 342) ausgebildete Steg (245') federnd-elastisch an der Wand des rückwärtigen Schalenteiles (220) gehalten ist.

17. Vorrichtung nach einem der Ansprüche 12 und 15,
dadurch gekennzeichnet,
daß der zwischen den schlitzförmigen Schenkelabschnitten (242, 342) ausgebildete Steg (245') über eine Gelenkverbindung mit der Wand des rückwärtigen Schalenteils (220) verbunden ist.

18. Vorrichtung nach einem der Ansprüche 12, 15 bis 17,
dadurch gekennzeichnet,
daß zur Fixierung der federnd-elastischen und gegeneinander verschiebbaren Wandabschnitte (225, 226, 245; 246, 346) in Anpassung an die jeweilige Wadenform und -größe des Unterschenkels das rückwärtige Schalenteil (220) benachbart zu seinem oberen Rand (220a) eine Anzahl von schlitzförmigen Durchbrechungen (250) zur Aufnahme eines Verschlußorganes, bevorzugterweise eines Spanngurtes oder -bandes (260), vorgesehen sind.

19. Vorrichtung nach einem der Ansprüche 12, 15 bis 18,
dadurch gekennzeichnet,
daß zur Fixierung der Wandabschnitte (225, 226) des rückwärtigen Schalenteils (220) in diesen benachbart zum oberen Schalenteilrand (220a) schlitzförmige Durchbrechungen (250) zur Aufnahme von Verschlußorganen, bevorzugterweise von Spanngurten oder -bändern (260) vorgesehen sind.

20. Vorrichtung nach Anspruch 19,
dadurch gekennzeichnet,
daß die Verschlußorgane an den Wandabschnitten (225, 226) des rückwärtigen Schalenteils (220) befestigt sind.

21. Vorrichtung nach Anspruch 13,
dadurch gekennzeichnet,
daß in die fensterartige Durchbrechung (280) im rückwärtigen Schalenteil (220) Winkeladapter (285) mit unterschiedlichen Längenanordnungen der Eingrifföffnungen (286; 286', 286'', 286''') für den Nocken (287) zur Winkeladapterfläche zur Fixierbarkeit des Fußteiles (222) zum Schaft (221) einsetzbar sind.

22. Vorrichtung nach einem der Ansprüche 13 und 21,
dadurch gekennzeichnet,
daß die fensterartige Durchbrechung (280) in er Wand des rückwärtigen Schalenteiles (220) etwa rechteckförmig ist und der Winkeladapter (285) eine der Form und den Abmessungen der fensterartigen Durchbrechung (280) entsprechende Ausgestaltung aufweist.

23. Vorrichtung nach einem der Ansprüche 13, 21 und 23,
dadurch gekennzeichnet,
daß die Länge der Eingrifföffnung (286) in dem Winkeladapter (285) einem einstellbaren Winkelbereich des Fußteils (222) zu dem Schaft (221) des rückwärtigen Schalenteiles (220) entspricht.

24. Vorrichtung nach einem der Ansprüche 13, 21 bis 23,
dadurch gekennzeichnet,
daß die Eingrifföffnung (286'') für den Nocken (287) im unteren Bereich des Winkeladapters (285) ausgebildet ist.

25. Vorrichtung nach einem der Ansprüche 13, 21 bis 24,
dadurch gekennzeichnet,
daß die Eingrifföffnung (286') für den Nocken (287) im oberen Bereich des Winkeladapters (285) ausgebildet ist.

26. Vorrichtung nach einem der Ansprüche 13, 21 bis 25,
dadurch gekennzeichnet
daß der Winkeladapter (285) eine sich über die Adapterlänge erstreckende schlitzförmige Eingrifföffnung (286''') für den Nocken (287) aufweist.

27. Vorrichtung nach einem der Ansprüche 13, 21 bis 26,
dadurch gekennzeichnet,
daß der plattenförmige Winkeladapter (285) an seinem umlaufenden Rand (285a) mit einer umlaufenden oder sich über sich gegenüberliegende Bereiche erstreckende Nuten (288) zum Eingriff des die fensterartige Durchbrechung (280) begrenzenden Randes (280a) versehen ist.

28. Vorrichtung nach einem der Ansprüche 13, 21 bis 27,
dadurch gekennzeichnet,
daß der plattenförmige Winkeladapter (285) aus einem federnd-elastischen Material, zweckmäßigerweise einem Kunststoff, besteht und mittels Klemmsitz am umlaufenden Rand (280a) der fensterartigen Durchbrechung (280) gehalten ist.

29. Vorrichtung nach einem der Ansprüche 13, 21 bis 28,
dadurch gekennzeichnet,
daß der plattenförmige Winkeladapter (285) an seinen beiden Längsseitenkanten (285b, 285c) keilförmig sich nach oben erweiternde Verstärkungen (289, 289a) aus einem federnd-elastischen Material aufweist.

30. Vorrichtung nach einem der Ansprüche 13, 21 bis 29,
dadurch gekennzeichnet,
daß der plattenförmige Winkeladapter (285) in seinem oberen Bereich zwei im Abstand voneinander angeordnete Durchbrechungen (283) zum Eingriff eines zangenartigen Öffnngswerkzeuges aufweist.

31. Vorrichtung nach einem der Ansprüche 13, 21 bis 30,
dadurch gekennzeichnet,
daß der Nocken (287) als Feststellschraube ausgebildet ist.

32. Vorrichtung nach einem der Ansprüche 1 bis 31,
dadurch gekennzeichnet,
daß das zum Schaft (231) verstellbare Fußteil (232) des vorderen Schalenteiles (230) über einen quer zur Schaftlängsrichtung verlaufenden ziehharmonikabalgartig ausgebildeten Abschnitt (291) mit einer Anzahl von gefalteten, filmscharnierartig miteinander verbundenen Materialstreifen (291a) verbunden ist, wobei die das Fußteil (232) und den Schaft (231) miteinander verbindenden seitlich an dem Schalenkörper (210) ausgebildeten Schwenkgelenke (235) mit Winkelarretierungseinrichtungen versehen sind.

33. Vorrichtung nach Anspruch 14,
dadurch gekennzeichnet,
daß die Winkeleinstellrichtung (290) aus einem zwischen dem Fußteil (232) und dem Schaft (231) des vorderen Schalenteils (230) angeordneten, dem Außenprofil des Schalenkörpers (210) bzw. des vorderen Schalenteils (230) entsprechend geformten plattenförmigen Winkeladapter (295) besteht, der mittels Rast- und Verriegelungseinrichtungen (297) an dem Schaft (231) und dem Fußteil (232) des vorderen Schalenteils (230) lösbar gehalten ist.

34. Vorrichtung nach einem der Ansprüche 14 und 33,
dadurch gekennzeichnet,
daß der plattenförmige Winkeladapter (295) eine Breite aufweist, aufgrund der das Fußteil (232) zu dem Schaft (231) des vorderen Schalenteils (230) eine Winkelstellung von 90° einnimmt.

35. Vorrichtung nach einem der Ansprüche 14, 33 und 34,
dadurch gekennzeichnet,
daß der plattenförmige Winkeladapter (295) eine Breite aufweist, aufgrund der das Fußteil (232) zu dem Schaft (231) des vorderen Schaltenteils (230) eine Winkelstellung von 120° einnimmt.

36. Vorrichtung nach einem der Ansprüche 14, 33 bis 35,
dadurch gekennzeichnet,
daß das Fußteil (232) und der Schaft (231) des vorderen Schalenteils (230) im Verbindungsbereich (239) eine festerartige Durchbrechung (292) aufweist, die sich bis in den Seitenbereich des Fußteiles (232) und des Schaftes (231) erstreckt und in der der plattenförmige Winkeladapter (295) angeordnet ist.

37. Vorrichtung nach einem der Ansprüche 14, 33 bis 36,
dadurch gekennzeichnet,
daß die gegenüberliegenden quer zur Schaftlängsrichtung verlaufenden Ränder (292a, 292b) der fensterartigen Durchbrechung (292) außenseitig wulstartige oder andersartig profilierte leistenförmige Verstärkungen (293) aufweisen und daß der plattenförmige Winkeladapter (295) an seinem oberen (295a) und unteren Rand (295b) mit den Verstärkungen (293) entsprechend ausgebildeten Gegenprofilen als Randprofile (296) versehen und mittels Klemmsitz an den Verstärkungen (293) an dem Fußteil (232) oder dem Schaft (231) des vorderen Schalenteils (230) gehalten ist.

38. Vorrichtung nach Anspruch 37,
dadurch gekennzeichnet,
daß die Randverstärkungen (293) an dem Fußteil (232) und dem Schaft (231) des vorderen Schalenteils (230) ein Querschnittsprofil mit einer quadratischen oder rechteckförmigen, konisch nach oben verlaufende Seitenflächen (293a, 293b) aufweisenden Fläche (293c) aufweisen, und daß die Randprofile (296) an dem plattenförmigen Winkeladapter (295) als entsprechendes, die Randverstärkungen (293) an dem Fußteil (232) und dem Schaft (231) übergreifendes Gegenprofil (296') ausgebildet sind.

39. Vorrichtung nach einem der Ansprüche 14, 33 bis 38,
dadurch gekennzeichnet,
daß zur Befestigung des plattenförmigen Winkeladapters (295) an dem Schaft (231) und dem Fußteil (232) des vorderen Schalenteils (230) der Winkeladapter (295) einendseitig (295c) und innenwandseitig mindestens zwei feststehende Verriegelungshaken (298), die in Durchbrechungen (298') in den Wandflächen des Schaftes (231) und des Fußteiles (232) eingreifen, und an seinem anderen Ende (295c) mindestens zwei drehbare als Exzenter ausgebildete Verriegelungshaken (299) aufweist, die in entsprechende Durchbrechungen (299') in den Wandflächen des Schaftes (231) und des Fußteiles (232) des vorderen Schalenteils (230) eingreifen.

40. Vorrichtung nach einem der Ansprüche 14, 33 bis 39,
dadurch gekennzeichnet,
daß der plattenförmige Winkeladapter (295) aus einem federnd-elastischen Material, zweckmäßigerweise aus einem Kunststoff besteht.

## Claims

1. External fixing device for extremities of bodies and extremity regions, more particularly for the treatment of extremity fractures within the area of the lower leg and thigh, comprised of a shell-type member (210) of a shell portion (220) or of two mutually tightenable shell portions (220, 230) which surround or accommodate the extremity to be ensheathed, in which, between the shell portions (220, 230) and the extremity, at least one sleeve-like padding (20) is provided, whose contents can be evacuated, in whose interior located between th two padding walls, a great number of loose filling material particles are provided which are moveable relative to each other, in which case the sleeve-like padding (20) possesses a plurality of intercommunicating vacuum-tight areas (400) filled with filling material particles, between which webs (420) and islands (420) having air passage apertures (411,421) are formed,
**characterized in that**
the sleeve-like padding (20), within the region of a muscle pad of the extremity to be ensheathed, preferably within the area of the calf of a lower leg, possesses a first insular area (430) that is provided with air passage apertures (431) and which is adjoined laterally and below by vacuum-tight areas (400) of the padding (20) and, within the area of the point where the heel is put on to the ground, possesses a second insular area (440) that is provided with air passage apertures (441) and which is surrounded by vacuum-tight areas (400) of the padding (20), by preference on both sides.

2. Device according to Claim 1,
**characterized in that**,
within the area of the foot and/or within the area of the Achilles tendon, an absorbent insole (20') is provided which, on its side pointing towards the extremity (E), possesses a layer (20'') which is semipermeable to vapour and/or moisture.

3. Device according to either Claim 1 or 2,
in which ventilation passages are provided in the foot portion,
**characterized in that**
a first shell portion (11) comprises a foot portion (10), in which a foot bedding (51) is disposed which possesses vertical ventilation passages (51') that lead into longitudinal ducts (51'') and/or transversal ducts (51'''), which are provided on the underside of the foot bedding (51).

4. Device according to Claim 3,
**characterized in that**
the transverse ducts (51''') proceed a long the outside of the foot bedding essentially in the upward direction and, by preference, open upwardly.

5. Device according to either Claim 3 or 4,
**characterized in that**
the transverse ducts (51''') are aligned with ventilation apertures (70) located in the foot portion (10).

6. Device according to any of Claims 1 to 5,
**characterized in that**
a first shell portion (11) comprises a foot portion (10) wherein a midsole (50) is disposed which, at its front end in the forward section of the foot portion (10), is swivellably supported about a transverse axis and which, within its rearward terminating region on the side of the heel, is engaged from below by a tightening element (72), by preference by a tightening strap, with the tightening strap proceeding within the area of transition from the foot portion to the leg portion and, on the outside, is mutually tightenable by means of the leg portion covering shell (12) engaging over the free rims of the leg portion and the foot portion.

7. Device according to Claim 6,
**characterized in that**,
on the outside, on the first shell portion (11), within the transitional area on the foot portion, closing or locking means (11') are provided which interact with the allocated closing or locking means (12') provided on the inside of the leg portion covering shell (12) in such a way that a clamping of the tightening means (72) brings about a reciprocal closing or locking of the closing or locking means (11', 12').

8. Device according to any of Claims 1 to 7,
**characterized in that**,
on the inside of the leg portion covering shell (11), hooking means for a padding (20), preferably essentially horizontally proceeding webs (11''), are constructed.

9. Device according to any of Claims 1 to 8,
**characterized in that**
both shell portions are comprised of plastic.

10. Device according to any of Claims 1 to 9,
**characterized in that**
the wall areas of each shell portion (220;230) are of solid wall construction.

11. Device according to any of Claims 1 to 10,
**characterized in that**
the wall areas of each shell portion (220;230) are constructed in a latticed manner.

12. Device according to any of Claims 1 to 11,
**characterized in that**,
of the two shell portions (220, 230) possessing the approximate configuration of an "L" and an approximately semicircular cross-sectional profile, the rearward shell, portion (220), while forming at least one bent-off section (245) issuing from the upper shell portion rim (220a), possesses slotted perforations (240, 340) that extend across an area in the longitudinal direction of the shell portion in such a way that, in the rearward shell portion (220), springably elastic and mutually displaceable wall sections (225, 226, 245) are formed and in that a means (260) fixating these sections (225, 226, 245) is provided.

13. Device according to any of Claims 1 to 12,
**characterized in that**,
of the two shell portions (220;230) possessing the approximate configuration of an "L" and an approximate semicircular cross-sectional profile and with a leg portion (221; 231) and a foot portion (222; 232), the rearward shell portion (220) possesses a foot portion (222) which is angularly adjustable relative to its leg portion (221) and, within the wall area located opposite the foot portion (222), a window-like perforation (80) with an interchangeable plate-shaped angle adapter (285) inserted into the latter, which is provided with an engagement recess or engagement opening (286), into which, for securing the angle between the foot portion (222) and the leg portion (22!) of the rearward shell portion (220), a lug (287) engages which, for the fixation of a predetrermined angular position is, within the rearward area of the foot portion, moulded on to the same.

14. Device according to any of Claims 1 to 13,
**characterized in that**,
of both shell portions (220, 230) possessing the approximate configuration of an "L" and an approximately semicircular cross-sectional profile and with a leg portion (221, 231) and a foot portion (222, 232), the front shell portion (230) possesses a foot portion (232) which is angularly adjustable relative to its leg portion (231) and, within the area between the foot portion (232) and the leg portion (231), possesses an angle adjusting means (290), with the aid of which the foot portion (232), in relation to the leg portion (231), is lockable in a predetermined angular position, while the rearward shell portion (220) possesses a foot portion (222) angularly adjustable relative to its leg portion (221) or a foot portion (222) fixed at a predetermined angle relative to its leg portion (221).

15. Device according to Claim 12,
**characterized in that**,
in the rearward shell portion (220), two slotted perforations (240, 340) proceeding in a spaced-apart manner in the longitudinal direction of the shell portion and extending from the upper shell rim (22a) are constructed which run out into approximately U-shaped sections (241, 341) possessing slotted leg sections (242, 342) proceeding parallel to each other so that, in the wall of the rearward shell portion (220), two tongue-like wall sections (246, 346) are formed relative to the wall sections (225, 226) adjacent to the slotted perforations (240, 340), in which case the wall sections (225, 226) adjoining the slotted perforations (240, 340) are fixed.

16. Device according to either Claim 12 or 15,
**characterized in that**
the web (245') formed between the slotted leg sections (242, 342) is springably resiliently retained on the wall of the rearward shell portion (220).

17. Device according to either Claim 12 or 15,
**characterized in that**
the web (245') formed between the slotted leg sections (242, 342) is connected by means of a hinge joint to the wall of the rearward shell portion (220).

18. Device according to any of Claims 12, 15 to 17,
**characterized in that**,
for the fixation of the springable elastic and mutually displaceable wall sections (225, 226, 245; 246, 346) in adaptation to the respective calf configuration and calf size of the lower leg, in the rearward shell portion (220), adjacent to its upper rim (220a), a plurality of slotted perforations (250) are provided for the accommodation of a closing means, by preference a tightening strap or band (260).

19. Device according to any of Claims 12, 15 to 18,
**characterized in that**,
for the fixation of the wall sections (225, 226) of the rearward shell portion (220), in the same, adjacent to the upper shell portion rim (220a), slotted perforations (250) are provided for the accommodation of closing means, preferably tightening straps or bans (260).

20. Device according to Claim 19,
**characterized in that**
the closing means are attached to the wall sections (225, 226) of the rearward shell portion (220).

21. Device according to Claim 13,
**characterized in that**,
into the window-like perforation (280) in the rearward shell portion (220), angle adapters (285) with different longitudinal arrangements of the engagement openings (286; 286', 286'', 286''') for the lug (287) relative to the angle adapter surface for the fixability of the foot portion (222) in relation to the leg portion (221) are insertable.

22. Device according to either Claim 13 or 21,
**characterized in that**
the window-like perforations (280) in the wall of the rearward shell portion (220) is approximately rectangular and in that the angle adapter (285) possesses a construction corresponding to the configuration and to the dimensions of the window-like perforation (280).

23. Device according to any of Claims 13, 21 and 23,
**characterized in that**
the length of the engagement opening (286) in the angle adapter (285) corresponds to an adjustable angular range of the foot portion (222) relative to the leg portion (221) of the rearward shell portion (220).

24. Device according to any of Claims 13, 21 to 23,
**characterized in that**
the engagement opening (286'') for the lug (287) is constructed within the lower area of the angle adapter (285).

25. Device according to any of Claims 13, 21 to 24,
**characterized in that**
the engagement opening (286') for the lug (287) is constructed within the upper area of the angle adapter (285).

26. Device according to any of Claims 13, 21 to 25,
**characterized in that**
the angle adapter (285) possesses a slotted angagemet opening (286''') for the lug (287) extending over the length of the adapter.

27. Device according to any of Claims 13, 21 to 26,
**characterized in that**
the plate-shaped angle adapter (285), on its circumferential edge (285a), is provided with a groove (288) which is either circumferential or extends across oppositely located areas for the engagement of the edge (280a) which delimits the window-like perforation (280).

28. Device according to any of Claims 13, 21 to 27,
**characterized in that**
the plate-shaped angle adapter (285) is composed of a springable elastic material, expediently a plastic and in that the same is retained by means of a press fit on the circumferential edge (280a) of the window-like perforation (280).

29. Device according to any of Claims 13, 21 to 28,
**characterized in that**,
the plate-shaped angle adapter (285), on its two longitudinal edges (285b, 285c), possesses reinforcing means (289, 289a) which expand upwardly in a wedge-like fashion and are comprised of a springable elastic material.

30. Device according to any of Claims 13, 21 to 29,
**characterized in that**,
the plate-shaped angle adapter (285), within its upper area, possesses two perforations (283) in a spaced-apart disposition for the engagement of a pliers-like opening tool.

31. Device according to any of Claims 13, 21 to 30,
**characterized in that**
the lug (287) is constructed in the form of a locking screw.

32. Device according to any of Claims 1 to 31,
**characterized in that**
the foot portion (232) which is adjustable in relation to the leg portion (231) of the front shell portion (230), by means of a section (291) proceeding transversally to the longitudinal direction of the leg portion which is constructed along the lines of an accordeon bellows, is connected with a plurality of folded, film hinge-like interconnected strips of material (291a), in which case the swivel hinges (235) which interconnect the foot portion (232) and the leg portion (231) constructed laterally on the shell member (210), are provided with angle locking means.

33. Device according to Claim 14,
**characterized in that**
the angle setting means (290) is comprised of a plate-shaped angle adapter (295) disposed between the foot portion (232) and the leg portion (231) of the front shell portion (230), configured so as to correspond to the external profile of the shell member (210) or of the front shell portion (230), which is detachably retained by means of snap-in connecting and closing means (297) on the leg portion (231) and on the foot portion (232) of the front shell portion (230).

34. Device according to either Claim 14 or 33,
**characterized in that**
the plate-shaped angle adapter (295) possesses a width, by virtue of which the foot portion (232), relative to the leg portion (231) of the front shell portion (230), assumes an angular position of 90°.

35. Device according to any of Claims 14, 33 and 34,
**characterized in that**
the plate-shaped angle adapter (295) possesses a width, by virtue of which the foot portion (232), relative to the leg portion (231) of the front shell portion (230), assumes an angular position of 120°.

36. Device according to any of Claims 14, 33 to 35,
**characterized in that**,
the foot portion (232) and the leg portion (231) of the front shell portion (230), within the connection area (239), possesses a window-like perforation (292) that extends as far as into the lateral area of the foot portion (232) and of the leg portion (231) and in which the plate-shaped angle adapter (295) is disposed.

37. Device according to any of Claims 14, 33 to 36,
**characterized in that**
the oppositely located rims (292a, 292b) proceeding transversally to the leg portion of the window-like perforation (292) possess, on their outside, bead-like or otherwise configured strip-like stiffeners (293) and in that the plate-shaped angle adapter (295), on its top (295a) and on its bottom edge (295b) with the stiffeners (293), is provided with pertinently constructed counter profile sections in the form of marginal profiles (296) and, by the means of press fit, is retained on the stiffeners (293) on the foot portion (232) or on the leg portion (231) of the front shell portion (230).

38. Device according to Claim 37,
**characterized in that**
the marginal stiffener elements (293) on the foot portion (232 and on the leg portioin (231) of the front shell portion (230) possess a cross-sectional profile with a surface (293c) having a square or rectangular, comically upwardly proceeding lateral surfaces (293a, 293b) and in that the marginal profiled (296) on the plate-shaped angle adapter (295) are constructed in the form of a corresponding counter profile section (296') engagimg over the marginal stiffening elements (293) on the foot portion (232) and on the leg portion (231).

39. Device according to any of Claims 14, 33 to 38,
**characterized in that**,
for the attachment of the plate-shaped angle adapter (295) on the leg portion (231) and on the foot portion (232) of the front shell, portion (230), the angle adapter (295), at one end (295c) and on the inner wall side, possesses at least two fixed closing hooks (298) which engage into perforations (298') in the wall areas of the leg portion (231) and of the foot portion (232) and, on its other end (295c), at least two rotatable closing hooks (299) constructed in the form of an eccentric, which engage into corresponding perforations (299') in the wall areas of the leg portion (231) and of the leg portion (231) and of the foot portion (232) of the front shell portion (230).

40. Device according to any of Claims 14, 33 to 39,
**characterized in that**
the plate-shaped angle adapter (295) is comprised of a springable elastic material, expediently of a plastic.

## Revendications

1. Dispositif pour la fixation enveloppante d'extrémités et de zones d'extrémités, en particulier pour le traitement des fractures des extrémités dans la zone de la jambe et de la cuisse, constitué par un corps en coque (210) constitué par une partie de coque (220) ou par deux parties de coque (220, 230) qui peuvent être serrées l'une contre l'autre, qui entourent ou logent l'extrémité à envelopper, au moins un coussin à manchette (20) dans lequel le vide peut être fait étant prévu entre les parties en coque (220, 230) et l'extrémité, coussin dans l'espace intérieur, situé entre les deux parois de coussin, duquel une multitude de corps de remplissage mobiles les uns par rapport aux autres sont prévus, le coussin à manchette (20) présentant une multitude de zones (400) étanches au vide, reliées l'une à l'autre et remplies de corps de remplissage, entre lesquelles des barrettes (410) et des îles (420) avec des ouvertures pour le passage de l'air (411, 421) sont formées,
caractérisé en ce
que le coussin à manchette (20) présente, dans la zone d'un bourrelet de muscle de l'extrémité à envelopper, de préférence dans la zone du mollet d'une jambe à envelopper, une première zone d'îles (430) qui est pourvue d'ouvertures pour le passage de l'air (431) et à laquelle des zones (400) du coussin (20), étanches au vide sur le côté et vers le bas, sont adjacentes et présente, dans la zone du point de contact du talon, une seconde zone d'îles (440), qui est équipée d'ouvertures pour le passage de l'air (441) et qui est entourée de zones étanches au vide (400) du coussin (20), de préférence de tous les côtés.

2. Dispositif selon la revendication 1,
caractérisé en ce
qu'un insert absorbant (20') est prévu dans la zone du pied et/ou dans la zone du tendon d'Achille, insert qui présente une couche semi-perméable (20'') pour la vapeur et/ou l'humidité sur le côté tourné vers l'extrémité (E).

3. Dispositif selon l'une des revendications 1 et 2, dans lequel des passages pour aération sont prévus dans la partie pied,
caractérisé en ce
qu'une première partie de coque (11) entoure une partie pied (10) dans laquelle un coussinet de pied (51) est placé qui présente des passages d'aération verticaux (51') qui débouchent dans des canaux longitudinaux (51'') et/ou des canaux transversaux (51''') qui sont prévus sur la face inférieure du coussinet de pied (51).

4. Dispositif selon la revendication 3,
caractérisé en ce
que les canaux transversaux (51''') sont essentiellement vers le haut le long des faces extérieures du coussinet de pied et qu'ils s'ouvrent de préférence vers le haut.

5. Dispositif selon l'une des revendications 3 et 4,
caractérisé en ce
que les canaux transversaux (51''') sont orientés avec des ouvertures d'aération (70) ménagées dans la partie pied (10).

6. Dispositif selon l'une des revendications 1 à 5,
caractérisé en ce
qu'une première partie de coque (11) entoure une partie pied (10) dans laquelle est placée une semelle intermédiaire (50) qui est logée sur sa face antérieure dans la section antérieure de la partie pied (10) en étant pivotante autour d'un axe transversal et qui est traversée par le bas, dans sa zone d'extrémité arrière côté talon, par un élément de serrage (72), de préférence une bande de serrage, la bande de serrage se trouvant dans la zone de la transition de la partie pied à la partie tige et pouvant être serrée à l'extérieur au-dessus de la coque de recouvrement de la tige (12) qui recouvre les bords libres de la partie tige et de la partie pied.

7. Dispositif selon la revendication 6,
caractérisé en ce
que des dispositifs d'enclenchement (11') sont prévus à l'extérieur sur la première partie de coque (11) dans la zone de la transition à la partie pied, dispositifs qui coopèrent avec des dispositifs d'enclenchement (12') correspondants, prévus à l'intérieur sur la coque de recouvrement de la tige (12), de telle manière qu'un serrage du dispositif de serrage (72) provoque un enclenchement mutuel du dispositif d'enclenchement (11', 12').

8. Dispositif selon l'une des revendications 1 à 7,
caractérisé en ce
que des dispositifs d'accrochage pour un coussin (20), de préférence des barrettes (11'') essentiellement horizontales, sont formés sur la face intérieure de la coque de recouvrement de la tige (11).

9. Dispositif selon l'une des revendications 1 à 8,
caractérisé en ce
que les deux parties de coque sont en matière plastique.

10. Dispositif selon l'une des revendications 1 à 9,
caractérisé en ce
que les surfaces de paroi de chacune des parties de coque (220 ; 230) sont formées à paroi pleine.

11. Dispositif selon l'une des revendications 1 à 10,
caractérisé en ce
que les surfaces de paroi de chacune des parties de coque (220 ; 230) sont formées en forme de grille.

12. Dispositif selon l'une des revendications 1 à 11,
caractérisé en ce
que, parmi les deux parties de coque (220, 230), qui présentent approximativement une forme de L avec un profil de section à peu près demi-circulaire, la partie de coque arrière (220) présente des découpures en forme de fentes (240, 340), qui partent du bord supérieur (220a) de la partie de coque, en formant au moins une section (245) que l'on peut courber, découpures qui s'étendent sur une zone dans le sens longitudinal de la partie de coque, de telle manière que des sections de paroi (225, 226, 245), élastiques à la manière d'un ressort et déplaçables l'une contre l'autre, sont formées dans la partie de coque arrière (220) et qu'un dispositif de fixation (260) de ces sections (225, 226, 245) est prévu.

13. Dispositif selon l'une des revendications 1 à 12,
caractérisé en ce
que, parmi les deux parties de coque (220 ; 230) qui présentent approximativement une forme de L avec un profil de section à peu près demi-circulaire et avec une tige (221 ; 231) et une partie pied (222 ; 232), la partie de coque arrière (220) présente une partie pied (222) dont l'angle est réglable par rapport à sa tige (221) et, dans la zone de paroi située en face de la partie pied (222), une découpure du type fenêtre (80) avec un adaptateur d'angle (285) remplaçable, en forme de plaque, mis en place dans celle-ci, qui est équipé d'un creux d'engrènement ou d'une ouverture d'engrènement (286) dans laquelle s'engrène une saillie (287) pour déterminer l'angle entre la partie pied (222) et la tige (221) de la partie de coque (220) arrière, saillie qui est moulée, pour fixer une position angulaire prédéterminée dans la zone arrière de la partie pied (22), sur celle-ci.

14. Dispositif selon l'une des revendications 1 à 13,
caractérisé en ce
que, parmi les deux parties de coque (220 ; 230) qui présentent approximativement une forme de L avec un profil de section à peu près demi-circulaire et avec une tige (221, 231) et une partie pied (222, 232), la partie de coque avant (230) présente une partie pied (232), dont l'angle est réglable par rapport à sa tige (231), et, dans la zone entre la partie pied (232) et la tige (231), un dispositif de réglage de l'angle (290) au moyen duquel la partie pied (232) peut être bloquée par rapport à la tige (231) dans une position angulaire prédéterminée, la partie de coque arrière (220) présentant une partie pied (222), dont l'angle est réglable par rapport à sa tige (221), ou une partie pied (222) fixée par rapport à sa tige (221) dans un angle prédéterminé.

15. Dispositif selon la revendication 12,
caractérisé en ce
que deux découpures en forme de fente (240, 340), qui s'étendent à partir du bord supérieur (22a) de la coque, dans le sens longitudinal de la partie de coque en étant écartées, sont formées dans la partie de coque arrière (220), découpures qui se terminent en sections approximativement en forme d'U (241, 341) avec des sections de montants (242, 342) en forme de fentes, parallèles l'une à l'autre, si bien que deux sections de paroi en forme de languette (246, 346) sont formées dans la paroi de la partie de coque arrière (220) en plus des sections de paroi (225, 226) voisines des découpures en forme de fente (240, 340), les sections de paroi (225, 226) voisines des découpures (240, 340) en forme de fente étant fixes.

16. Dispositif selon l'une des revendications 12 et 15,
caractérisé en ce
que la barrette (245') formée entre les sections de montants (242, 342) en forme de fentes est maintenue de manière élastique à la manière d'un ressort sur la paroi de la partie de coque arrière (220).

17. Dispositif selon l'une des revendications 12 et 15,
caractérisé en ce
que la barrette (245') formée entre les sections de montant (242, 342) en forme de fentes est reliée par une jonction articulée à la paroi de la partie de coque arrière (220).

18. Dispositif selon l'une des revendications 12, 15 à 17,
caractérisé en ce
que, pour la fixation des sections de paroi (225, 226, 245 ; 246, 346) élastiques à la manière d'un ressort et déplacables l'une contre l'autre en adaptation à la forme du mollet respectif et à la taille de la jambe, la partie de coque arrière (220) présente, au voisinage de son bord supérieur (220a), un certain nombre de découpures en forme de fentes (250) pour loger un organe de fermeture, de préférence une sangle de serrage ou une bande de serrage (260).

19. Dispositif selon l'une des revendications 12, 15 à 18,
caractérisé en ce
que, pour a fixation des sections de paroi (225, 226) de la partie de coque arrière (220), des découpures en forme de fentes (250) sont prévues dans cette partie de coque au voisinage du bord supérieur (220a) de la partie de coque pour loger des organes de fermeture, de préférence des sangles de serrage ou des bandes de serrage (260).

20. Dispositif selon la revendication 19,
caractérisé en ce
que les organes de fermeture sont fixés sur les sections de paroi (225, 226) de la partie de coque arrière (220).

21. Dispositif selon la revendication 13,
caractérisé en ce
que des adaptateurs d'angle (285) avec différents arrangements en longueur des ouvertures d'engrènement (286 ; 286', 286'', 286''') peuvent être mis en place dans la découpure du type fenêtre (280) dans la partie de coque arrière (220) pour la saillie (287) pour la surface de l'adaptateur d'angle pour la fixabilité de la partie pied (222) sur la tige (221).

22. Dispositif selon l'une des revendications 13 et 21,
caractérisé en ce
que la découpure du type fenêtre (280) dans la paroi de la partie de coque arrière (220) est à peu près rectangulaire et que l'adaptateur d'angle (285) présente une configuration qui correspond à la forme et aux dimensions de la découpure du type fenêtre (280).

23. Dispositif selon l'une des revendications 13, 21 et 23,
caractérisé en ce
que la longueur de l'ouverture d'engrènement (286) dans l'adaptateur d'angle (285) correspond à une zone angulaire réglable de la partie pied (222) par rapport à la tige (221) de la partie de coque arrière (220).

24. Dispositif selon l'une des revendications 13, 21 à 23,
caractérisé en ce
que l'ouverture d'engrènement (286'') pour la saillie (287) est formée dans la zone inférieure de l'adaptateur d'angle (285).

25. Dispositif selon l'une des revendications 13, 21 à 24,
caractérisé en ce
que l'ouverture d'engrènement (286') pour la saillie est formée dans la zone supérieure de l'adaptateur d'ange (285).

26. Dispositif selon l'une des revendications 13, 21 à 25,
caractérisé en ce
que l'adaptateur angulaire (285) présente une ouverture d'engrènement (286''') en forme de fente qui s'étend sur la longueur de l'adaptateur pour la saillie (287).

27. Dispositif selon l'une des revendications 13, 21 à 26,
caractérisé en ce
que l'adaptateur angulaire en forme de plaque (285) est pourvu, sur son bord périphérique (285a), d'une rainure périphérique ou de rainures qui s'étendent sur des zones opposées l'une à l'autre pour l'engrènement du bord (280a) qui délimite la découpure du type fenêtre (280).

28. Dispositif selon l'une des revendications 13, 21 à 27,
caractérisé en ce
que l'adaptateur angulaire en forme de plaque (285) est constitué par une matière élastique comme un ressort, de manière appropriée par une matière plastique, et est maintenu par ajustement pressé sur le bord périphérique (280a) de la découpure de type fenêtre (280).

29. Dispositif selon l'une des revendications 13, 21 à 28,
caractérisé en ce
que l'adaptateur angulaire en forme de plaque (285) présente, sur ses deux arêtes de bords longitudinaux (285b, 285c), des renforcements (289, 289a), qui s'élargissent en coin vers le haut, en une matière élastique comme un ressort.

30. Dispositif selon l'une des revendications 13, 21 à 29,
caractérisé en ce
que l'adaptateur angulaire en forme de plaque (285) présente, dans sa zone supérieure, deux découpures (283) placées à un certain écart l'une de l'autre pour l'engrènement d'un outil d'ouverture de type pince.

31. Dispositif selon l'une des revendications 13, 21 à 30,
caractérisé en ce
que la saillie (287) est configurée comme une vis de blocage.

32. Dispositif selon l'une des revendications 1 à 31,
caractérisé en ce
que la partie pied (232) de la partie de coque avant (230), qui est réglable par rapport à la tige (231), est reliée, par une section (291) configurée comme un soufflet d'harmonica transversalement par rapport au sens longitudinal de la tige, à un certain nombre de bandes de matière (291a) pliées, reliées l'une à l'autre à la manière d'une charnière en film, les articulations pivotantes (235) formées sur le côté du corps de coque (210) et qui relient la partie pied (232) et la tige (231) l'une à l'autre étant équipées de dispositifs de blocage d'angle.

33. Dispositif selon la revendication 14,
caractérisé en ce
que le dispositif de réglage d'angle (290) est constitué par un adaptateur d'angle (295) en forme de plaque, placé entre la partie pied (232) et la tige (231) de la partie de coque avant (230), formé de manière correspondante au profil extérieur du corps de coque (210) ou de la partie de coque avant (230), adaptateur d'angle qui est maintenu amovible au moyen de dispositifs d'enclenchement et de verrouillage (297) sur la tige (231) et la partie pied (232) de la partie de coque avant (230).

34. Dispositif selon l'une des revendications 14 et 33,
caractérisé en ce
que l'adaptateur d'angle en forme de plaque (295) a une largeur en raison de laquelle la partie pied (232) prend une position angulaire de 90° par rapport à la tige (231) de la partie de coque avant (230).

35. Dispositif selon l'une des revendications 14, 33 et 34,
caractérisé en ce
que l'adaptateur d'angle en forme de plaque (295) a une largeur en raison de laquelle la partie pied (232) prend une position angulaire de 120° par rapport à la tige (231) de la partie de coque avant (230).

36. Dispositif selon l'une des revendications 14, 33 à 35,
caractérisé en ce
que la partie pied (232) et la tige (231) de la partie de coque avant (230) présente une découpure de type fenêtre (292) dans la zone de jonction (239), découpure qui s'étend jusque dans la zone latérale de la partie pied (232) et de la tige (231) et dans laquelle l'adaptateur d'angle en forme de plaque (295) est placé.

37. Dispositif selon l'une des revendications 14, 33 à 36,
caractérisé en ce
que les bords (292a, 292b) de la découpure de type fenêtre (292), qui sont opposés transversalement par rapport au sens longitudinal de la tige, présentent des renforcements (293) de type bourrelet sur la face extérieure ou en forme de baguettes profilées d'un autre type et que l'adaptateur en forme de plaque (295) est pourvu son bord supérieur (295a) et sur son bord inférieur (295b) de profilés antagonistes comme profilés de bord (296) formés de manière à correspondre aux renforcements (293) et est maintenu par ajustement pressé sur les renforcements (293) de la partie pied (232) ou de la tige (231) de la partie de coque avant (230).

38. Dispositif selon la revendication 37,
caractérisé en ce
que les renforcements de bord (293) sur la partie pied (232) et la tige (231) de la partie de coque avant (230) présentent un profil de section avec une surface (293c) carrée ou rectangulaire qui présente des faces latérales (293a, 293b) coniques vers le haut et que les profils de bord (296) sont formés sur l'adaptateur d'angle en forme de plaque (295) comme profil antagoniste (296') corespondant qui recouvre les renforcements de bord (293) sur la partie pied (232) et la tige (231).

39. Dispositif selon l'une des revendications 14, 33 à 38,
caractérisé en ce
que, pour la fixation de l'adaptateur d'angle en forme de plaque (295) sur la tige (231) et la partie pied (232) de la partie de coque avant (230), l'adaptateur d'angle (295) présente, à l'une de ses extrémités (295c) et sur la face de la paroi intérieure, au moins deux crochets de verrouillage (298) fixes qui s'engrènent dans des découpures (298') dans les faces de paroi de la tige (231) et de la partie pied (232) et, à son autre extrémité (295c), au moins deux crochets de verrouillage (299) rotatifs, configurés comme des excentriques, qui s'engrènent dans des découpures correspondantes (299') dans les faces de paroi de la tige (231) et de la partie pied (232) de la partie de coque avant (230).

40. Dispositif selon l'une des revendications 14, 33 à 39,
caractérisé en ce
que l'adaptateur d'angle en forme de plaque (295) est constitué par une matière élastique comme un ressort, de manière approprée par une matière plastique.
